# EUROPEAN PATENT SPECIFICATION

(11) **EP 1 938 761 B1**
(45) Date of publication and mention of the grant of the patent: **15.03.2017**
(21) Application number: 06810782.0
(22) Date of filing: 28.09.2006
(51) Int. Cl.: A61B 17/00, A61B 17/04, A61B 17/02, A61B 17/06

(54) **SUTURING DEVICE**
NAHTVORRICHTUNG
DISPOSITIF DE SUTURE

(30) Priority: 28.09.2005 US 238016
(43) Date of publication of application: 02.07.2008
(73) Proprietor: Olympus Corporation, Hachioji-shi, Tokyo 192-8507 (JP)
(72) Inventor: MIKKAICHI, Takayasu, Fuchu-shi, Tokyo 183-0033 (JP); IWASAKA, Masayuki, Tama-shi, Tokyo 206-0034 (JP); SUZUKI, Takayuki, Yokohama-shi, Kanagawa 226-0027 (JP); KAJI, Kunihide, Hachioji-shi, Tokyo 192-0023 (JP); SHIONO, Junji, Yokohama-shi Kanagawa 226-0011 (JP); HAYASHI, Kensuke, Kanagawa-ken (JP); SATO, Masatoshi, Yokohama-shi Kanagawa 226-0011 (JP)
(74) Representative: von Hellfeld, Axel
(86) International application number: PCT/JP2006/319335
(87) International publication number: WO 2007/037335

(56) References cited:
- WO-A1-99/21490
- WO-A2-2005/079673
- JP-A- 10 504 222
- JP-A- 2005 211 690
- US-A- 4 235 238
- US-A- 5 624 380
- US-A1- 2003 045 778
- US-A1- 2003 216 613
- US-A1- 2004 138 525
- US-A1- 2004 225 305

## Description

### [TECHNICAL FIELD]

The present invention relates to a suture instrument that inserts a suture tool through the mouth or anus and sutures a perforation. For example, it relates to a suture instrument that sutures a perforation that is formed in the wall of a hollow organ.

### [BACKGROUND ART]

In the case of performing treatment in a body of a patient, the treatment can be performed by incising the body of the patient by surgical operation, or by oral endoscopic treatment or transanal endoscopic treatment. A method for suturing a perforation formed in an abdominal area by surgical operation is disclosed in FIGS. 6a to 6c of U.S. Patent No. 6,066,146. According to this suturing method, a needle is thrust into the tissue around the perforation, and an anchor equipped with a suture thread is then extruded from the needle. After the needle is drawn out from the tissue, two suture threads across the perforation are knotted together to close the perforation.

The treatment using an endoscope is carried out by passing a forceps, high-frequency treatment instrument, incision instrument, suture tool or the like through a channel of the endoscope. When the medical treatment is carried out by using an endoscope inserted into a hollow organ through a natural opening of a living body such as the mouth, anus, or the like, for example, a hole is formed by removing the tissue from the abdominal cavity or incising the tissue in the abdominal cavity, and the medical treatment is then carried out by approaching the abdominal cavity through this hole from the inside of the hollow organ. After performing the medical treatment, the formed hole is sutured by a suture tool.

Here, a method for suturing in a hollow organ is disclosed in FIGS. 6 to 9 of Japanese Unexamined Patent Application, First Publication No. 2004-601, for example. According to this suturing method, the tissue is drawn into an overtube, and a needle is then thrust through this tissue from the proximal side to the distal end side thereof. From the inside of the needle, an anchor equipped with a suture thread is pushed out to the distal end side of the tissue. After that, when the needle is pulled out, the suture thread penetrates through the tissue, and so the tissue is tightened up by this suture thread. There is also a method disclosed in FIG. 1, FIG. 4, FIGS. 5A to 5C of U.S. Patent No. 5,297,536. According to this method, a flexible endoscope is inserted into the vicinity of a perforation via the mouth or the anus. The tissue around the perforation is aspirated by a tube of the flexible endoscope. When an O-ring provided at the outside of the tube is pushed out from the distal end of the tube, the aspirated tissue is clamped by the O-ring.
Patent Document 1: U.S. Patent No. 6,066,146

WO 2005/079673 A2, upon which the preamble of claim 1 is based, discloses a delivery system, which is incorporated with an articulating endoscope. The endoscope includes an elongated body with the capability of articulation having a proximal end and a distal end. A delivery tube having a flexible elongated body with a proximal end and a distal end and a central lumen extending at least partially between the proximal end and the distal ends, is disposed within a lumen of the endoscope. The delivery tube includes an election port at the distal end. The endoscope is then articulated to direct the distal end of the delivery tube toward the portion of the stomach wall where the placement of a stricture is desired. The endoscope is articulated so that its distal end is curved to face the stomach wall. After an anchor is secured in the stomach wall, the endoscope can be twisted or rotated for the placement of the next anchor.

WO 99/21490 A1 discloses a device for passing attachment apparatus or connector materials into or through a wall of a luminal anatomical structure. The device comprises a transluminal catheter, a slotted hollow needle, which can be advanced out of the catheter. The hollow needle is formed of a super elastic material. A sharpened distal tip of the needle will puncture fully or partially through the wall of the anatomical conduit. The needle may be deflected and is constructed to carry a plurality of attachment members. Each attachment member comprises a pliable link formed of a suture threat. Engagement members are formed upon opposite ends of each link. The engagement member remains within a lumen of the needle and remains coupled to any subsequent engagement members which are also within the needle lumen.

US2004/0225305 A1, upon which the preamble of claim 7 is based, discloses an apparatus for forming gastrointestinal tissue folds with adjustable anchor assemblies.

US5624380 discloses a multi-degree-of-freedom manipulator having a plurality of flex portions and a plurality of actuators made of shape memory alloy for flexing the flex portions.

US2004/0138525 A1 discloses an endoscopic tool for deploying a fixation device such as an anchor which will hold tissue plication in place. The tool has a polymer wall coextruded with shape memory material, such as nitinol wire.

### [DISCLOSURE OF THE INVENTION]

### [PROBLEM TO BE SOLVED BY THE INVENTION]

When passing the suture thread through tissue around the perforation, it is necessary to confirm there are no other internal organs and the like in that vicinity. However, confirming other internal organs with a conventional suture instrument has been difficult. Here, when passing the needle through the tissue around the perforation from the outer side to the inner side, it is possible to readily confirm that the needle has penetrated the tissue, however, with a conventional suture instrument, it has not been possible to readily pass a needle from the outer side to the inner side in the state of being protruded to the outer side of an organ through punched holes.

The present invention was achieved in view of the above circumstances, and has as its main object to be able to reliably pass a suture tool that sutures a perforation from the outer side to the inner side.

### [DISCLOSURE OF THE INVENTION]

The invention is defined in the appended independent claim 1 with an alternative solution to the same problem in independent claim 7.

Preferred embodiments are described in the dependent claims.

In the suture instrument in accordance with the first aspect of the present invention, when the anchor holding instrument is passed through a perforation and placed on the outer side, by driving the tip position changing mechanism with the position operation device, the anchor holding instrument is moved in a direction that intersects with the axial line in the lengthwise direction of the base. When the base is pulled back in this state, since the anchor holding instrument passes through the tissue around the perforation from the outer side to the inner side, when the anchors are pushed out from the tip of the anchor holding instrument, it is possible to eject the anchors in the state of the suture thread passing through the tissue.

A second aspect of the present invention is the suture instrument in accordance with the first aspect, wherein the tip position changing mechanism changes the position by causing the anchor holding instrument to move to a position that is offset from the axial line in the lengthwise direction of the base.

In the suture instrument in accordance with the second aspect of the present invention, by offsetting the anchor holding instrument with respect to the axial line of the base, it is possible to point the tip of the anchor holding instrument toward the tissue around the perforation.

A third aspect of the present invention is the suture instrument in accordance with the first aspect, wherein the tip position changing mechanism changes the position by inclining the anchor holding instrument with respect to the axial line in the lengthwise direction of the base.

In the suture instrument in accordance with the third aspect of the present invention, by inclining the anchor holding instrument, it is possible to point the tip of the anchor holding instrument toward the tissue around the perforation.

A fourth aspect of the present invention is the suture instrument in accordance with any one of the first aspect to the third aspect, wherein the anchor holding instrument is a needle that has at the distal end a sharp tip.

In the suture instrument in accordance with the fourth aspect of the present invention, since it is possible to pierce the tissue with the anchor holding instrument, there is no need to form a hole in the tissue in advance with another treatment tool or the like.

A fifth aspect of the present invention is the suture instrument in accordance with any one of the first aspect to the third aspect, wherein the distal end of the anchor holding instrument consists of a hollow needle, and the anchors are disposed in the needle.

In this suture instrument in accordance with the fifth aspect of the present invention, when having pierced through the tissue with the anchor holding instrument, it is possible to push out and eject the anchors as is.

A sixth aspect of the present invention is the suture instrument in accordance with any one of the first aspect to the fifth aspect, further provided with an attraction instrument that attracts the anchors with a magnetic force, wherein the base has a lumen, the attraction instrument is disposed along the base so as to move freely forward or backward, and the anchors and the attraction instrument are both made from a magnetic body, and one of them is a magnet.

In this suture instrument in accordance with the sixth aspect of the present invention, when the attraction instrument is brought near an anchor, the anchor is ejected from the anchor holding instrument.

The above-mentioned object is alternatively also attained with a suture instrument having the features set out in independent claim 7.

In the suture instrument in accordance with the seventh aspect of the present invention, when the anchor holding instrument is passed through a perforation and placed on the outer side, by driving the deflection mechanism with the position operation device, the orientation of the anchor holding instrument is made to point to the proximal end side of the base. When the base is pulled back in this state, since the anchor holding instrument passes through the tissue around the perforation from the outer side to the inner side, when the anchors are pushed out from the tip of the anchor holding instrument, the anchors are placed in the state of the suture thread passing through the tissue.

An eighth aspect of the present instrument is the suture instrument in accordance with the seventh aspect, wherein the tip position changing mechanism changes the position by inclining the anchor holding instrument with respect to the axial line in the lengthwise direction of the base.

In this suture instrument in accordance with the seventh aspect of the present invention, by inclining the anchor holding instrument, it is possible to point the distal end of the anchor holding instrument to the tissue around the perforation.

A ninth aspect of the present invention is the suture instrument in accordance with the seventh aspect or the eighth aspect, wherein the anchor holding instrument is a needle that has at the distal end a sharp tip.

In this suture instrument in accordance with the ninth aspect of the present invention, since it is possible to pierce through the tissue with the anchor holding instrument, there is no need to form a hole in the tissue in advance with another treatment tool or the like.

A tenth aspect of the present invention is the suture instrument in accordance with the seventh aspect or the eighth aspect, wherein the distal end of the anchor holding instrument consists of a hollow needle, and the anchors are disposed in the needle.

In this suture instrument in accordance with the tenth aspect of the present invention, when having pierced through the tissue with the anchor holding instrument, it is possible to push out and eject the anchors as is.

An eleventh aspect of the present invention is the suture instrument in accordance with any one of the seventh aspect to the tenth aspect, further provided with an attraction instrument that attracts the anchors with a magnetic force, wherein the base has a lumen, the attraction instrument is disposed along the base so as to move freely forward or backward, and the anchors and the attraction instrument are both made from a magnetic body, and one of them is a magnet.

In this suture instrument in accordance with the eleventh aspect of the present invention, when the attraction instrument is brought near an anchor, the anchor is ejected from the anchor holding instrument.

### [EFFECT OF THE INVENTION]

In accordance with the present invention, since it is possible to deploy the anchor holding instrument to make it point toward tissue around a perforation in the state of the base being passed through the perforation, if the anchor holding instrument is moved toward the tissue in this state, it is possible to make it pass from the outer side to the inner side of the tissue. Accordingly, it is possible to eject the anchors from the outer side to the inner side of the tissue with a simple operation.

### [BRIEF DESCRIPTION OF DRAWINGS]

FIG 1 is a view that shows a schematic constitution of an endoscope and a suture instrument.
FIG 2 is a cross-sectional view of a suture instrument and the distal end portion of an endoscope.
FIG. 3 is a perspective view of a suture instrument and the distal end portion of an endoscope.
FIG 4 is a view that shows the constitution of a suture tool.
FIG 5 is a schematic view that shows a step of inserting an endoscope into the stomach of a patient to observe a perforation from within the stomach.
FIG 6 is a schematic view showing a step of observing the outer side of the stomach.
FIG 7 is a schematic view showing a step of puncturing the tissue with a needle of a suture instrument.
FIG 8 is a schematic view showing a step of pushing an anchor out from a needle to the outer side of the stomach.
FIG. 9 is a schematic view in which two anchors are placed outer side of the stomach.
FIG. 10 is a schematic view showing a step of tightening up a perforation by a suture tool.
FIG. 11 is a schematic view that describes the operation for grasping a suture tool with a forceps.
FIG. 12 is a view in which a perforation is sutured by a forceps and a suture tool.
FIG 13 is a schematic view showing a rod which is an example of an excluding device.
FIG 14 is a schematic view showing a balloon catheter which is an example of an excluding device.
FIG 15 is a schematic view showing a balloon catheter in which a balloon is inflated.
FIG 16 is a schematic view showing a forceps which is an example of an excluding device.
FIG 17 is a schematic view showing one example of a combination of an endoscope with a suture instrument.
FIG. 18 is a schematic view showing one example of a combination of an endoscope with a suture instrument.
FIG 19 is a schematic view showing one example of a combination of an endoscope using an overtube with a suture instrument.
FIG 20 is a schematic view showing one example of a combination of an endoscope with an observation device.
FIG 21 is a schematic view showing one example of a combination of an endoscope with a suture instrument.
FIG 22 is a schematic view showing one example of a combination of an endoscope using an overtube with a suture instrument.
FIG 23 is a schematic view showing a step of observing the outer side of the stomach.
FIG. 24 is a schematic view showing a step of puncturing the tissue from the outer side of the stomach with a needle of a suture instrument.
FIG 25 is a schematic view showing a step of pushing out an anchor from a needle to the inner side of the stomach.
FIG 26 is a schematic view in which two anchors are placed on the outer side of the stomach.
FIG 27 is a schematic view showing a step of tightening up a perforation with a suture tool.
FIG 28 is a schematic view describing the operation of grasping a suture tool by a forceps.
FIG. 29 is a view in which a perforation is sutured by a forceps and a suture tool.
FIG 30 is a schematic view showing a step of observing the outer side of the stomach.
FIG. 31 is a schematic view showing a step of puncturing the tissue with a needle of a suture instrument.
FIG. 32 is a schematic view showing a step of pushing out an anchor from a needle to the inner side of the stomach.
FIG 33 is a schematic view in which two anchors are placed on the outer side of the stomach.
FIG 34 is a schematic view showing a step of tightening up a perforation with a suture tool.
FIG. 35 is a schematic view describing a method of thrusting a needle from the inner side of the stomach after observing the inner side and the outer side of the stomach by an endoscope.
FIG 36 is a schematic view showing one example of a combination of an endoscope with a suture instrument.
FIG 37 is an enlarged view of the constitution of the distal end portion of the suture instrument.
FIG 38 is a view from arrow A in FIG. 37 that shows the constitution of the distal end portion of the suture instrument.
FIG 39 is a view that describes the constitution of the operation unit of the suture instrument that is passed through the endoscope.
FIG. 40 is a view that shows the state of the pair of forceps pieces of the suture instrument being opened and describes a procedure.
FIG. 41 is a view of piercing through the tissue from the outer side to the inner side with a needle and pushing out and ejecting a first anchor.
FIG. 42 is a view of piercing through the tissue on the opposite side centered on the perforation with the needle and pushing out and ejecting a second anchor.
FIG 43 is an enlarged view of the distal end portion of the suture instrument that is provided with a needle that freely rotates.
FIG. 44 is a view that describes the constitution of the operation unit of the suture instrument that is passed through the endoscope.
FIG 45 is a view that shows the state of opening the needle and describes a procedure piercing the needle through tissue.
FIG 46 is a view that describes the operation of bringing the pusher toward the needle that has been pierced through the tissue and pulling out an anchor.
FIG 47 is a view that describes the operation of attracting and pulling an anchor onto the pusher.
FIG. 48 is a view of pulling away and releasing the anchor from the pusher.
FIG 49 is an enlarged view of the distal end portion of a suture instrument in which the needle is curved in an approximate U-shape.
FIG 50 is a view of pulling out and deploying the needle from the second lumen.
FIG 51 is a view of protruding the needle to the outer side from the perforation in the state of being housed in the second lumen.
FIG 52 is a view of pulling out the needle from the second lumen and deploying it to the tissue.
FIG 53 is an enlarged view of the distal end portion of the suture instrument that has a curving portion that changes the direction of the needle.
FIG 54 is a view of the curving portion being curved.
FIG 55 is an enlarged view of the distal end portion of the suture instrument that has a balloon at the distal end portion of the sheath.
FIG 56 is a view that shows the sheath being curved by swelling the balloon.
FIG 57 is a cross-sectional view along line B-B in FIG 56.
FIG 58 is a view that shows the sheath being curved by swelling the balloon and an outside organ being excluded.
FIG 59 is an enlarged view of the distal end portion of the suture instrument that has an erecting hook, with the distal end portion of the sheath curved approximately 90 degrees.
FIG 60 is a view that describes the constitution of an operation unit of the suture instrument that is passed through the endoscope.
FIG. 61 is a view that describes the operation of catching the tissue around a perforation by the erecting hook.
FIG. 62 is a view of pulling the suture instrument and drawing in the tissue that is caught by the erecting hook, and piercing the needle through the tissue.
FIG 63 is a view showing a cross-section of the distal end portion of the suture instrument that does not have a needle.
FIG. 64 is a perspective view of the distal end portion of the suture instrument shown in FIG 63.
FIG. 65 is a view that shows a high-frequency knife as an example of a treatment tool to be used together with the suture instrument.
FIG 66 is a view that explains a procedure, and is a view of forming a small incision hole with a high-frequency knife.
FIG. 67 is a view of FIG 66 seen from the side.
FIG 68 is a view that describes a procedure of curving an outer sheath of the suture instrument to pass through a small incision hole.
FIG 69 is a view of pushing out and ejecting an anchor to the inside of the stomach.
FIG 70 is a view for explaining the order of tightening a plurality of suture tools.

### [BRIEF DESCRIPTION OF THE REFERENCE NUMERALS]

13 inner sheath (base, anchor holding instrument)
16 suture tool
17, 121, 145, 190 operation unit (position operation device)
27, 141, anchor
42 perforation
101, 131, 151, 161, 171, 181 suture instrument
102, 152, 162 sheath (base)
103 treatment portion (tip position changing mechanism)
112, 139, 163, 183, 193 needle (anchor holding instrument)
112A, 139A, 163A sharp tip
106 second forceps piece (opening-closing member)
134 lumen
140 operating wire (tip position changing mechanism)
153, 184 first lumen
154 second lumen
163A tip
164 curving portion (deflection mechanism)
168 curving control member (shape-memory alloy)
173 balloon
182 sheath (position operation device)
188 erecting hook

### [BEST MODE FOR CARRYING OUT THE INVENTION]

Next, each embodiment of the present invention shall be described with reference to the drawings. Note that in the descriptions of each embodiment, the same reference numbers shall be given to identical portions. Also, descriptions of overlapping portions shall be omitted.

### [First Embodiment not forming part of the present invention]

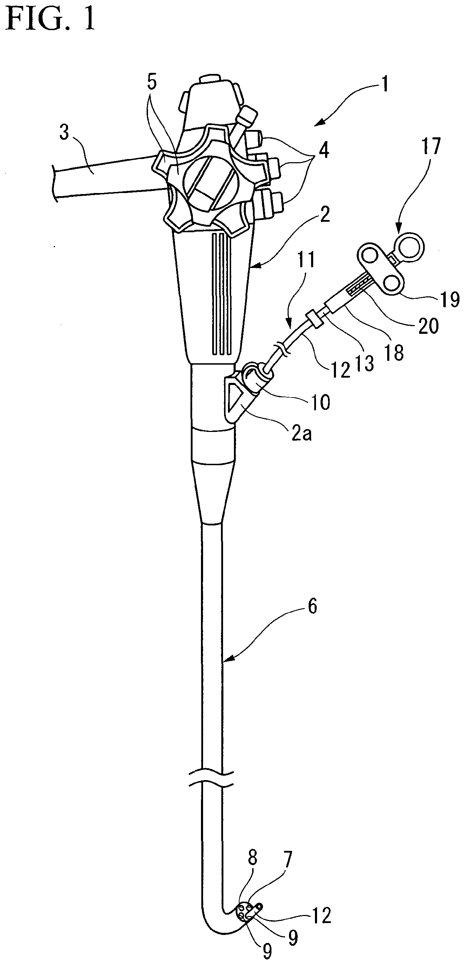

In FIG 1, an endoscope and a suture instrument used in this embodiment are shown. An endoscope 1 (flexible endoscope) has an endoscope operation unit 2 which is operated by an operator. The endoscope operation unit 2 is connected to a control device via a universal cable 3 and equipped with various switches 4 and angle knobs 5. At the distal end of the endoscope operation unit 2, an endoscope insertion part 6 that is flexible and long is extendedly formed. At the distal end of the endoscope insertion part 6, an endoscope observation device (a first observation device, hereinafter simply referred to as an observation device) 7 which obtains an image of the internal body, a lighting unit 8, and a distal end opening of a channel 9 are provided. As the observation device 7, an imaging device having a CCD (Charge Coupled Device), an optical fiber, and the like can be used. The lighting unit 8 has an optical fiber that conducts light from a light source. The channel 9 opens at a lateral part 2a of the endoscope operation unit 2 through the endoscope insertion part 6. At an opening of the lateral part 2a, a cap 10 is provided. In the cap 10, an insertion hole is formed, and a treatment tool such as a suture instrument 11 or an observation device is inserted into the channel 9 through this insertion hole.

As shown in FIGS. 1 to 3, in the suture instrument 11, an inner sheath 13 which is a flexible base is passed through the inside of a flexible outer sheath 12 so as to be able to freely move forward or backward. At the remote end separated from the hand side (that is, the distal end) of the inner sheath 13, a hollow needle 14 having a sharp tip is fixed. A slit 15 extends in the lengthwise direction of the needle 14 from the distal end thereof. A suture tool 16 is contained inside of the outer sheath 12 and the needle 14. Each of the lengths of the outer sheath 12 and the inner sheath 13 is longer than that of the channel 9 of the endoscope 1. At the proximal end on a hand side of the inner sheath 13 (hereinbelow, the base end), an operation unit 17 is provided.

The operation unit 17 has a handle 19 which can freely slide with respect to a main body 18 of the operation unit. To the handle 19, a base end of a pusher 20 is fixed. The pusher 20 extends through the inside of the inner sheath 13 to the inside of the needle 14. A distal end portion 21 of the pusher 20 is pressed against an anchor 27 of the suture tool 16.

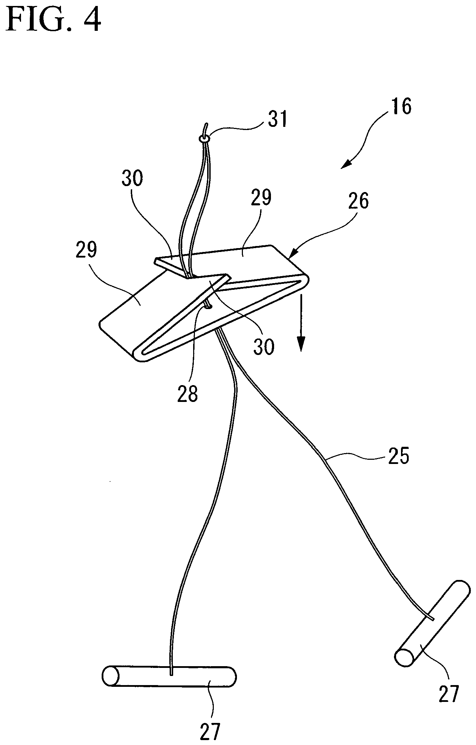

As shown in FIG 4, the suture tool 16 has a suture thread 25. The suture thread 25 is folded approximately in two and a knot 31 is formed in the vicinity of its turn-around point. Moreover, the suture thread 25 is bundled at both end portions thereof and passed through a stopper 26 that is substantially triangular. To each end portion of the suture thread 25, an anchor 27 is fixed. The anchor 27 has a cylindrical shape and the suture thread 25 is fixed at the approximately center portion in a longitudinal direction of the anchor 27. The stopper 26 includes a long, thin plate member in which a hole 28 is formed at the center portion in a longitudinal direction thereof, through which the suture thread 25 is passed. Both end portions 29 in a longitudinal direction of the stopper 26 are diagonally folded back to hold the suture thread 25 therebetween. Both end portions 29 in a longitudinal direction of the stopper 26 are cut to form triangular sections 30. Both end portions 29 of the stopper 26 are diagonally folded back so that the sections 30 intersect with each other to hold the suture thread 25 therebetween. As a result, the suture thread 25 is prevented from passing through a space formed between end portions 29. When the knot 31 of the suture thread 25 is pulled in a direction away from the stopper 26, both end portions 29 of the stopper 26 are slightly opened. Accordingly, the stopper 26 allows the suture thread 25 to move in this direction. On the other hand, when end portions of the suture thread 25 at the side of the anchors 27 are pulled, the suture thread 25 is ready to move in a direction shown by an arrow in FIG 4. However, both end portions 29 of the stopper 26 close and secure the suture thread 25 at this time, and thereby the suture thread 25 does not move.

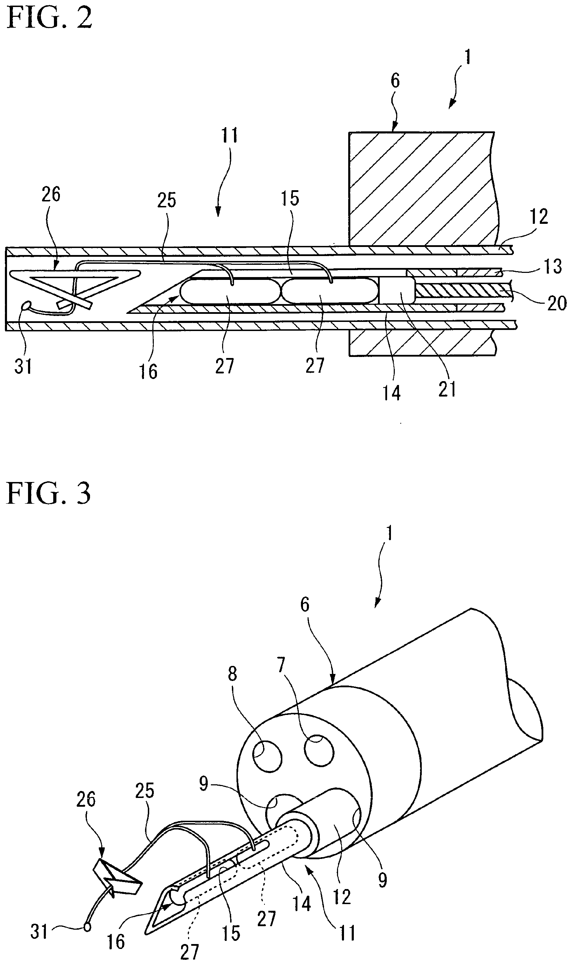

As shown in FIG 3, the suture tool 16 sequentially houses two anchors 27 in an inner hole of the needle 14 that functions as an anchor holding instrument. The suture thread 25 is drawn out from the slit 15 of the needle 14. As shown in FIG 2, the stopper 26 is held at a more distal end portion than the needle 14 in the outer sheath 12. The number of the anchors 27 and the shape of the stopper 26 are not limited to the embodiment shown in the figures.

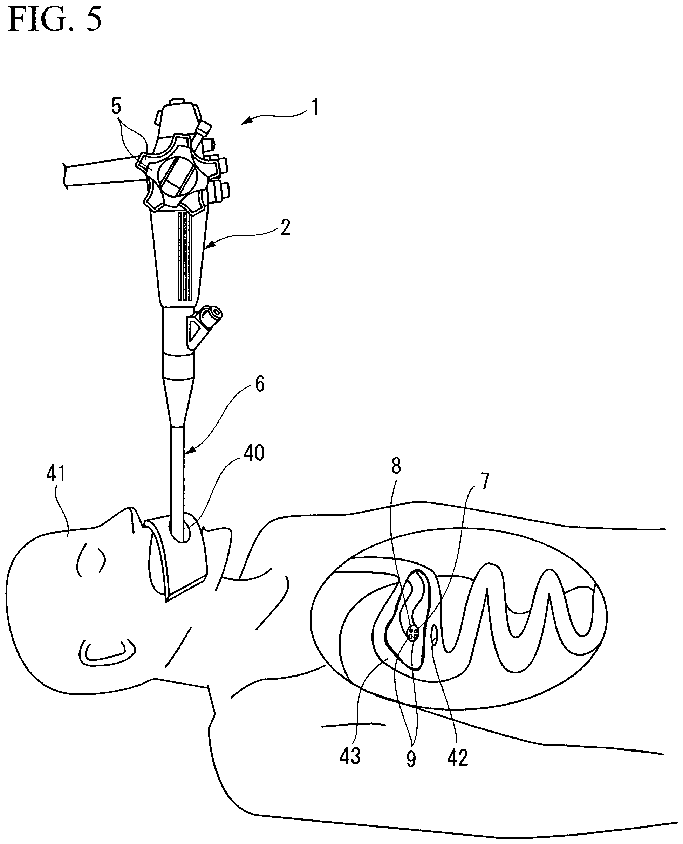

Next, a suturing method of this embodiment will be explained mainly with reference to FIGS. 5 to 12. FIGS. 5 to 12 are pattern diagrams illustrating procedure and show the stomach as an example of a hollow organ.

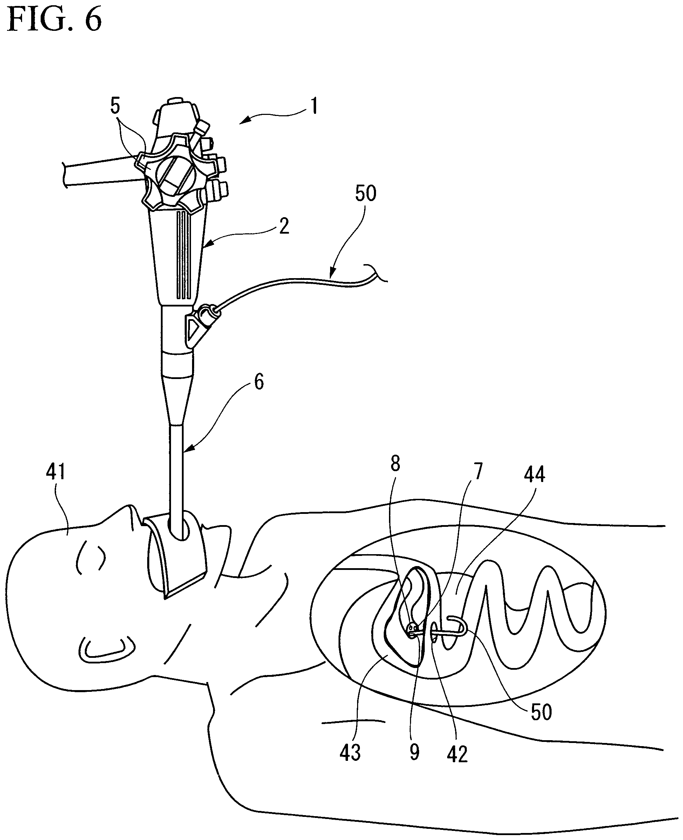

As shown in FIG. 5, the endoscope insertion part 6 is inserted from the mouth (including a natural opening of a living body, such as the anus, nose, or ear) of a patient 41 prepared with a mouthpiece 40. When the tip of the endoscope insertion part 6 is bent by the angle knob 5, a perforation 42 can be checked by the observation device 7 from the inside of the stomach 43 (the inside of the hollow organ). As shown in FIG 6, an observation device (second observation device) 50 is passed through the channel 9 of the endoscope 1. The observation device 50 is, for example, a catheter having a camera at the tip thereof. The observation device 50 may be a long and narrow fiberscope.

The distal end of the observation device 50 is inserted into an abdominal cavity 44 from the perforation 42, and the distal end of the observation device 50 is bent back by a wire or the like which is not shown in the figures. By using the observation device 50, an area around the perforation 42 to be punctured with the needle 14 (referred also to as a puncture position or a position through which the needle 14 passes) is observed from an abdominal cavity 44 side (which is also the side at which the anchor 27 is placed), that is, from the outer side of the stomach 43 (referred also as a body cavity side of the hollow organ or the abdominal cavity side) to check that other tissues such as the small intestine, the liver, or the like do not exist at the position through which the needle 14 is passed in order to prevent these tissues from being punctured or sutured together.

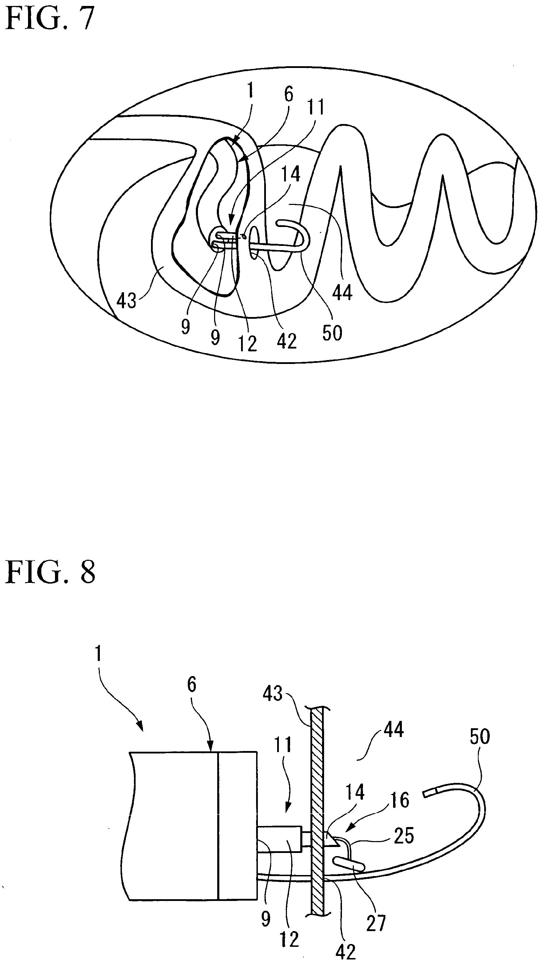

As shown in FIG 7, the suture instrument 11 is projected to puncture the tissue around the perforation 42 with the needle 14 while observing the stomach 43 from the abdominal cavity 44 side by the observation device 50. When the tissue is punctured, the needle 14 is projected from the outer sheath 12 as shown in FIG. 3. In the process of projecting the needle 14, the stopper 26 which is contained at the more distal end portion than the needle 14 is extruded from the outer sheath 12 into the stomach 43. When the needle 14 is moved forward with the outer sheath 12 fixed, the needle 14 punctures the tissue. When the handle 19 provided at an operator-side as shown in FIG 1 is pushed in, the pusher 20 moves forward, and the first anchor 27 is pushed out from the tip of the needle 14 into the abdominal cavity 44, as shown in FIG 8. When the first anchor 27 is pushed out and released, the pusher 20 is stopped, and the needle 14 is drawn out from the tissue. The first anchor 27 remains on the abdominal cavity 44 side. The suture thread 25 penetrates through the tissue. Note that the stopper 26 exists inside of the stomach 43.

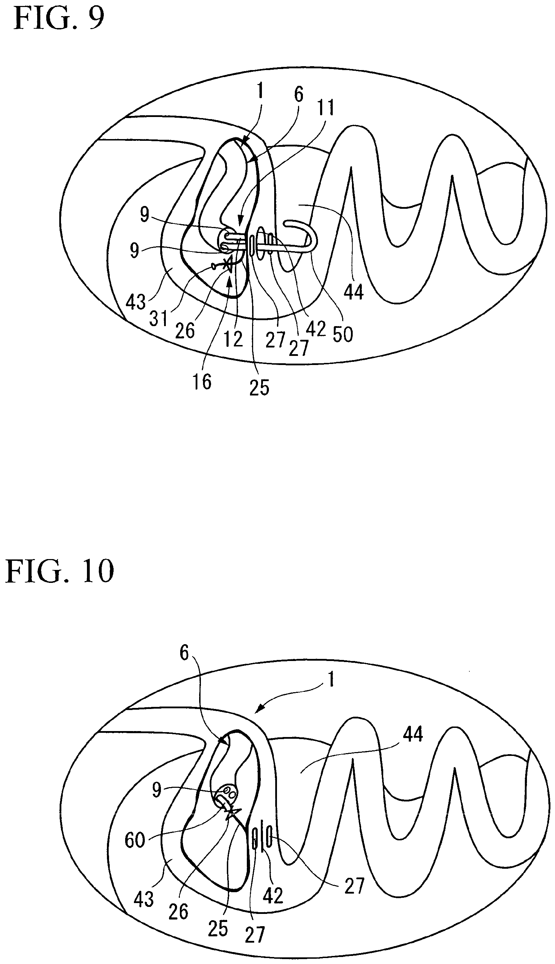

Moreover, the needle 14 is thrust again at an approximately symmetrical position with respect to the position at which the needle 14 is previously thrust centered about the perforation 42. In the same manner as when using the first anchor 27, when the needle 14 penetrates through the tissue, the pusher 20 is moved forward. The second anchor 27 is pushed out into the abdominal cavity 44. As shown in FIG 9, when the needle 14 is drawn back, the second anchor 27 remains on the abdominal cavity 44 side, the suture thread 25 penetrates through the tissue, and the second anchor 27 is released to the side of the abdominal cavity 44 so as to sandwich the perforation 42 therebetween.

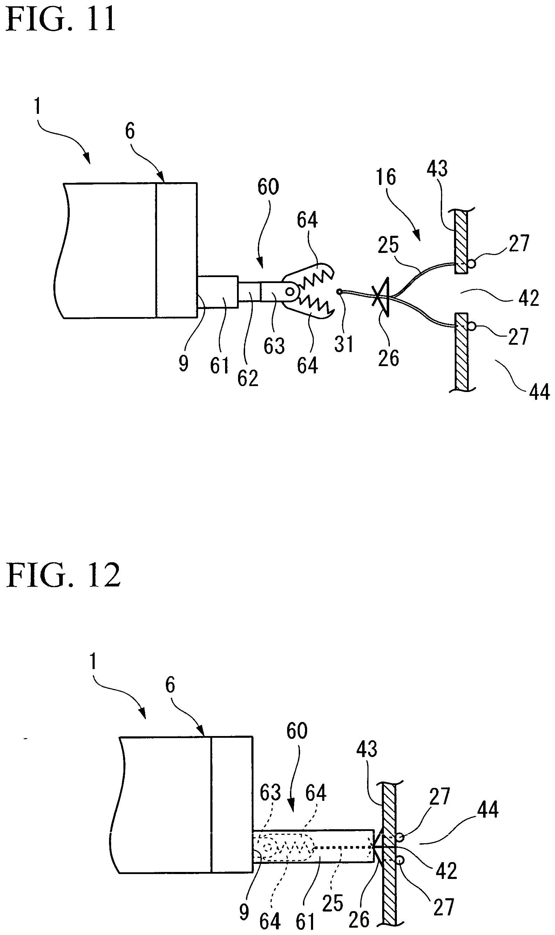

Next, as shown in FIG. 10, after the observation device 50 is drawn back to the inside of the stomach 43, the suture thread 25 is pulled so that the anchor 27 and the stopper 26 tighten up the tissue, and thereby the perforation 42 is sutured. When the suture thread 25 is pulled, a forceps 60 shown in FIG. 11, for example, is used. The forceps 60 is passed through the channel 9 in the place of the observation device 50. The forceps 60 has an outer sheath 61 having an external diameter larger than the anchor 27 and an inner sheath 62 passed through the outer sheath 61 so as to freely move forward or backward. At the tip of the inner sheath 62, a supporting member 63 is provided, and a pair of grip segments 64 is supported on the supporting member 63 so as to freely open or close.

After the knot 31 of the suture thread 25 of the suture tool 16 is gripped by the grip segments 64, the outer sheath 61 is moved forward to press the tip of the outer sheath 61 against the stopper 26. As shown in FIG. 12, when the outer sheath 61 moves further forward, the stopper 26 is pushed into the wall of the stomach 43. Since the stopper 26 is constructed to be able to move in this direction, the stopper 26 moves toward the wall. Since the position of the pair of the grip segments 64 does not change, the stopper 26 moves relatively forward with respect to the suture thread 25. As a result, the distance between the stopper 26 and the anchor 27 decreases. This pulls together the tissue around the perforation 42, and the perforation 42 is sutured by the suture thread 25. After suturing the perforation 42 by the suture tool 16, the outer sheath 61 is moved backward, and the grip segments 64 are then opened to release the suture thread 25. Although the tip of the stopper 26 can move in a direction in which the tissue is tightened up by the suture thread 25, it acts to tighten up the suture thread 25 in a direction for loosening the suture thread 25. As a result, the suture thread 25 is not loosened, even if the suture tool 16 is placed inside of the stomach 43.

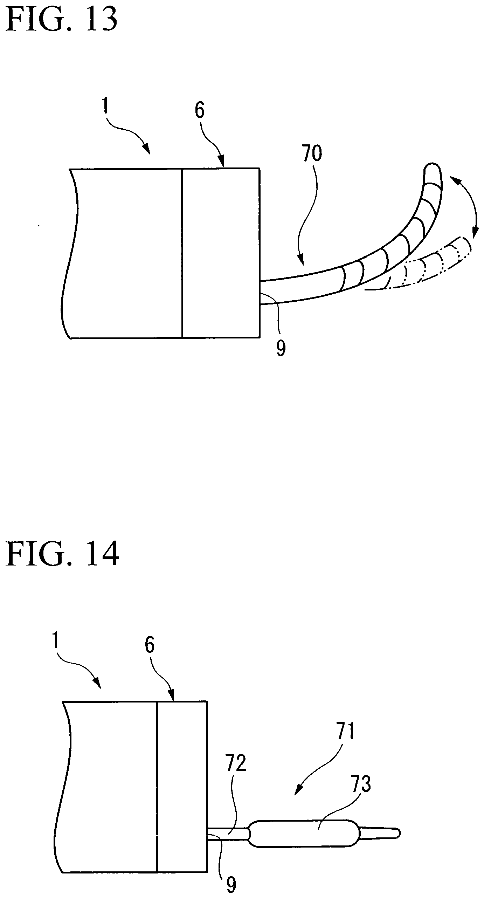

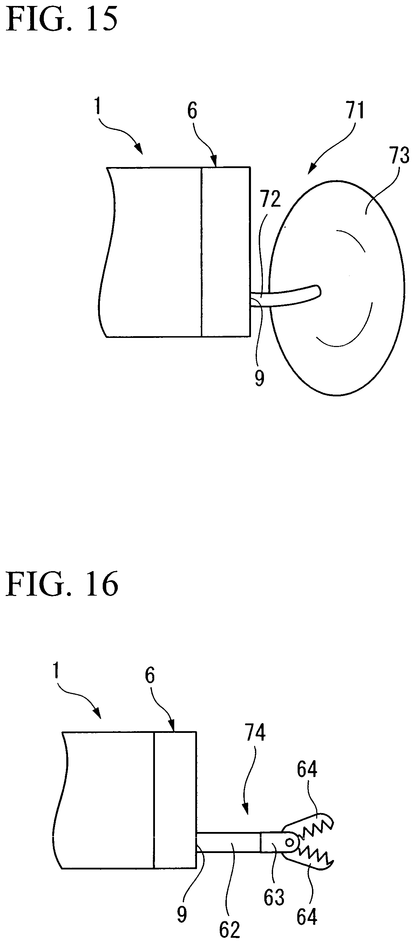

When a hollow organ such as the small intestine large intestine or another organ such as the spleen or the liver (hereinafter, merely referred to as tissue) exists around the perforation 42 (the position through which the needle 14 is passed), the other tissue is excluded from the stomach 43 by inserting an excluding device. The excluding device used in this case is exemplified in FIGS. 13 to 16. An excluding device shown in FIG 13 is a rod 70 of which a tip portion can be bent. When the rod 70 is bent, the other hollow organ is excluded to form a space through which the needle 14 is passed. An excluding device shown in FIGS. 14 and 15 is a balloon catheter 71. When a balloon 73 provided at the tip portion of a catheter 72 is inflated by supplying a fluid from the operator-side to exclude the other hollow organ, the space through which the needle 14 is passed is formed. An excluding device shown in FIG 16 is a forceps 74. When the other hollow organ is grasped by the forceps 74 to draw it away from the stomach 43, the space through which the needle 14 is passed is formed. At the tip portions of these excluding devices, an optical fiber or an observation device having a CCD may be provided. When the observation device is provided, it becomes possible to exclude other tissues while observing the state of the abdominal cavity 44.

In this embodiment, the perforation 42 is observed from the inside of the stomach 43 by the observation device 7 of the endoscope 1 at first, and the perforation 42 is then observed from the abdominal cavity 44 side by the observation device 50. After that, the suture instrument 11 is made to penetrate through the tissue around the perforation 42 to mount the suture tool 16, and the perforation 42 is sutured by using this suture tool 16. Accordingly, it is possible to suture the perforation 42 after respectively checking from the inside (the side from which the needle 14 is thrust) and the outside (the side through which the needle 14 penetrates or at which the anchor 27 is placed) of the stomach 43 that another tissue does not exist around the perforation 42. According to a suturing method using an endoscope of the prior art, it is impossible to check the opposite side. According to the endoscopic suturing method in this embodiment, it is possible to easily and reliably check for the existence of other tissues, as a result of which procedure can be carried out with rapidity.

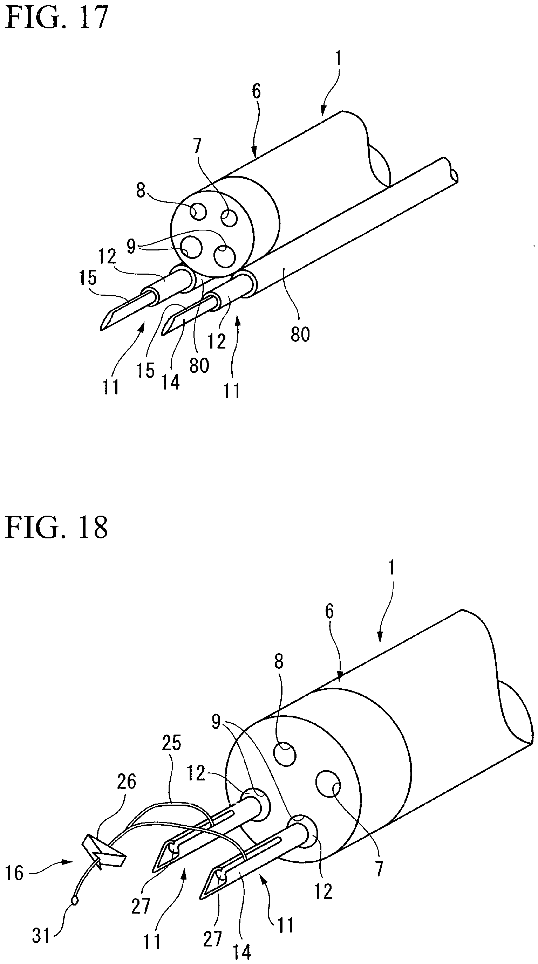

Modified examples of this embodiment are shown in FIGS. 17 to 22.

As shown in FIG. 17, two external sheaths 80 are provided at the periphery of the endoscope insertion part 6. A suture instrument 11 is passed through each external sheath 80 so as to freely move forward or backward. The anchors 27 are individually contained in the respective needles 14. It is possible to thrust two needles 14 into the tissue at the same time or in an arbitrary order. As another example, one external sheath 80 may be used, and two anchors 27 may be contained in one needle 14. Moreover, FIG. 18 shows an example in which the suture instruments 11 are individually passed through two channels 9 of the endoscope insertion part 6.

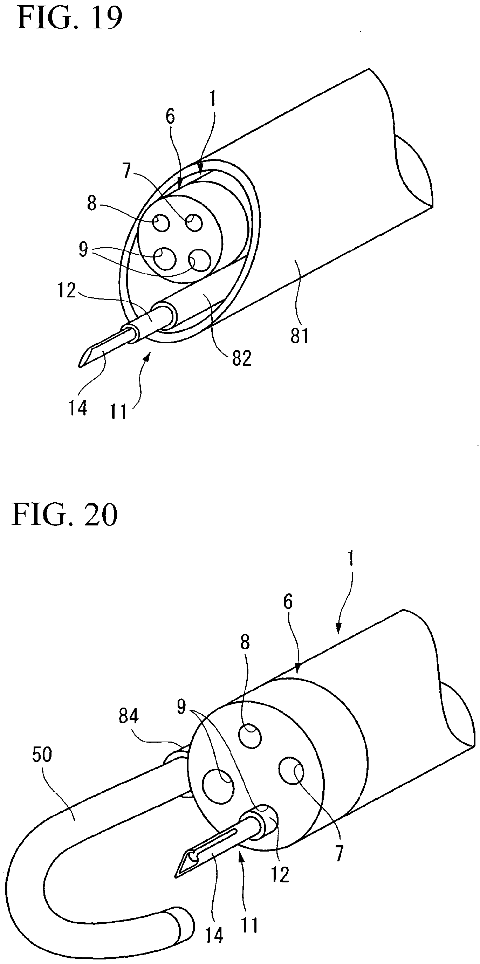

As shown in FIG 19, the endoscope insertion part 6 is inserted into an overtube 81. At the inner periphery of the overtube 81, a lumen 82 is provided, and the suture instrument 11 is passed through the lumen 82. At the inner periphery of the overtube 81, two lumens 82 may be provided, and the suture instruments 11 may be individually passed through each of the lumens 82.

As shown in FIG 20, a channel 84 may be provided at the periphery of the endoscope insertion part 6, and the observation device 50 may be passed through this channel 84. Moreover, the observation device 50 may be directly provided at the periphery of the endoscope insertion part 6 without using the channel 84.

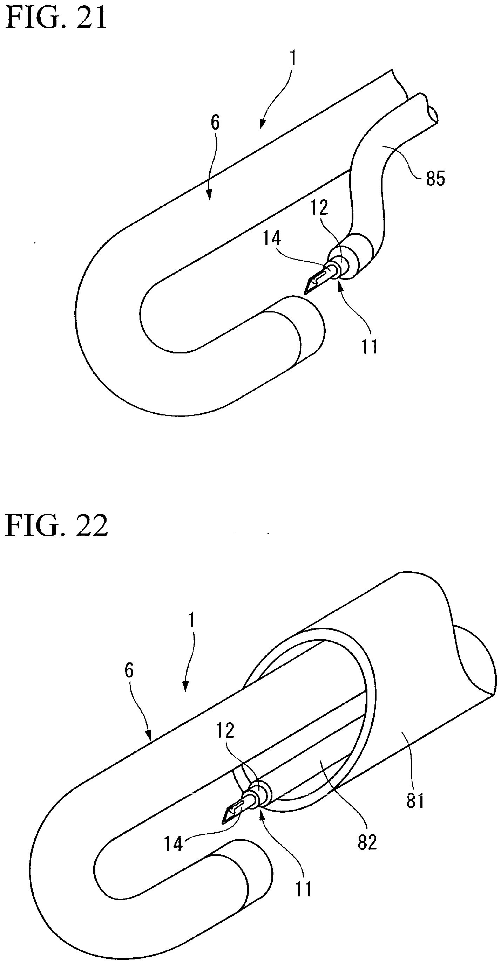

As shown in FIG 21, an external channel 85 may be provided parallel to the endoscope insertion part 6, and the suture instrument 11 may be passed through the channel 85. The tip portion of this channel 85 can be bent. When observing the perforation 42 from the abdominal cavity 44 side as shown in FIG 6, the endoscope insertion part 6 is passed through the perforation 42 and moved into the abdominal cavity 44, and the tip portion of the endoscope insertion part 6 is then bent to observe by the observation device 7 provided at the tip portion thereof.

As shown in FIG 22, the suture instrument 11 may be passed through the lumen 82 formed inside of the overtube 81. In this case, the area around the perforation 42 is observed from the abdominal cavity 44 side by using the observation device 7 of the endoscope insertion part 6.

### [Second Embodiment not forming part of the present invention]

In this embodiment, the same endoscope 1 and suture instrument 11 as in the first embodiment are used. Descriptions that overlap with the first embodiment will be omitted.

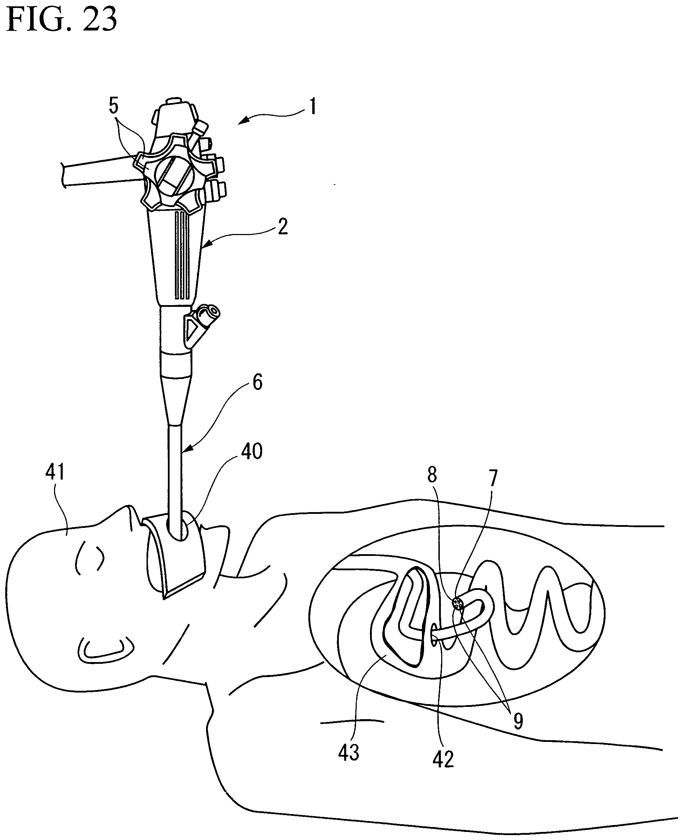

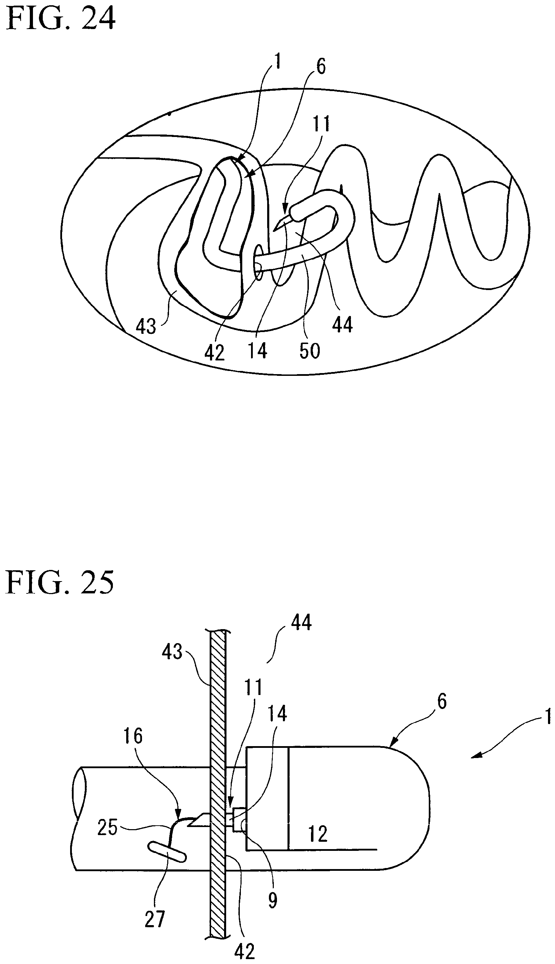

A suturing method of this embodiment will be explained. As shown in FIG. 5, the endoscope insertion part 6 is inserted into the vicinity of the perforation 42 to observe the perforation 42 from the inside of the stomach 43. Next, as shown in FIG. 23, the endoscope insertion part 6 is moved from the perforation 42 into the abdominal cavity 44, and an area around the perforation 42 is then observed from the abdominal cavity 44 side by the observation device (first observation device) 7 of the endoscope insertion part 6. After confirming that other hollow organs do not exist in the area around the perforation 42, the needle 14 of the suture instrument 11 is projected from the endoscope insertion part 6 as shown in FIG 24, and the needle 14 is thrust from the abdominal cavity 44 side into the stomach 43. Since the safety of the inside of the stomach 43 is confirmed first, the inside of the stomach 43 may not be checked when thrusting the needle 14. The inside of the stomach 43, however, may be punctured while observing the inside of the stomach 43 (the side at which the anchor 27 is placed) by using another observation device. In this case, it is possible to puncture at the puncture position while observing from both the inner side of the stomach 43 and the abdominal cavity 44 side.

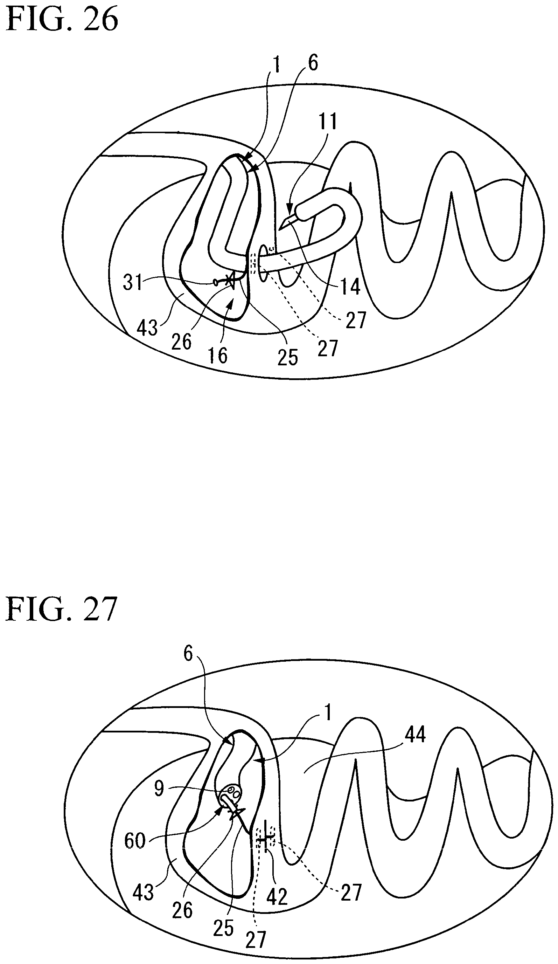

As shown in FIG. 25, the first anchor 27 is extruded into the stomach 43 from the tip of the needle 14. As shown in FIG. 26, after placing two anchors 27 so as to sandwich the perforation 42 therebetween, the suture instrument 11 is contained inside of the channel 9. After that, the endoscope 1 is drawn back to the inside of the stomach 43.

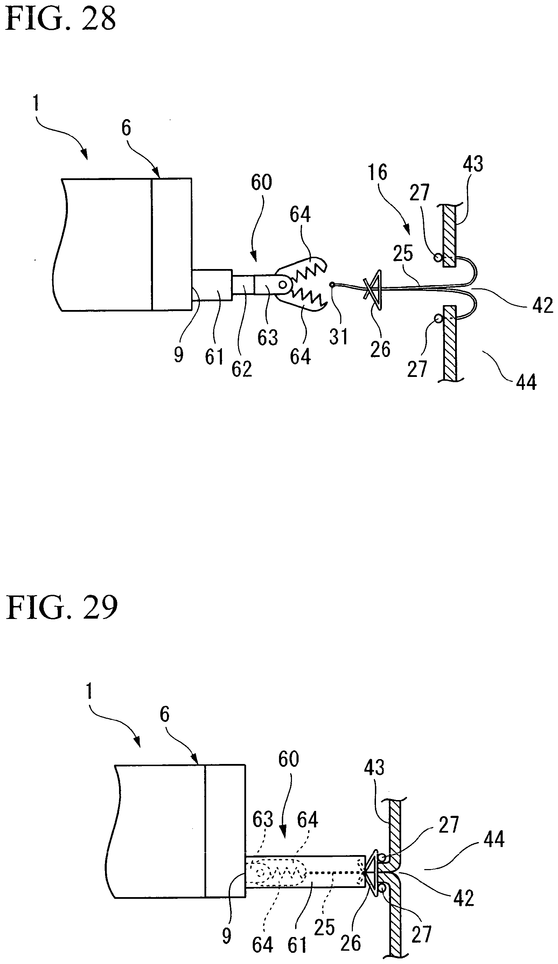

As shown in FIGS. 27 and 28, the forceps 60 is passed through the channel 9 of the endoscope 1 drawn back to the inside of the stomach 43. The forceps 60 grasps the knot 31 of the suture thread 25 existing in the abdominal cavity 44 side, and draws the suture thread 25 and the stopper 26 into the inside of the stomach 43 through the perforation 42. As shown in FIG. 29, when the stopper 26 is pressed against the tissue by the outer sheath 61, the suture tool 16 tightens up the tissue, and thereby the perforation 42 is sutured.

In this embodiment, after observation of the inner side of the stomach 43 by using the endoscope 1, the endoscope 1 is moved to the outer side of the stomach 43 to check by the observation device 7 from the abdominal cavity 44 side that other tissues do not exist in the area around the perforation 42. After that, the needle 14 is thrust into the tissue from the outer side to mount the suture tool 16 and suture the perforation 42 while maintaining the endoscope 1 passing through the perforation 42. Accordingly, other tissues can be easily prevented from being sutured together when suturing by using the endoscope 1.

### [Third Embodiment not forming part of the present invention]

In this embodiment, the same endoscope 1 and suture instrument 11 as in the first embodiment are used. Descriptions that overlap with the first embodiment will be omitted.

A suturing method of this embodiment will be explained. As shown in FIG. 5, the endoscope insertion part 6 is inserted in the vicinity of the perforation 42 to observe the perforation 42 from the inside of the stomach 43. Next, as shown in FIG. 23, the endoscope insertion part 6 is moved from the perforation 42 into the abdominal cavity 44, and an area around the perforation 42 is then observed from the abdominal cavity 44 side by the observation device (first observation device) 7 of the endoscope insertion part 6.

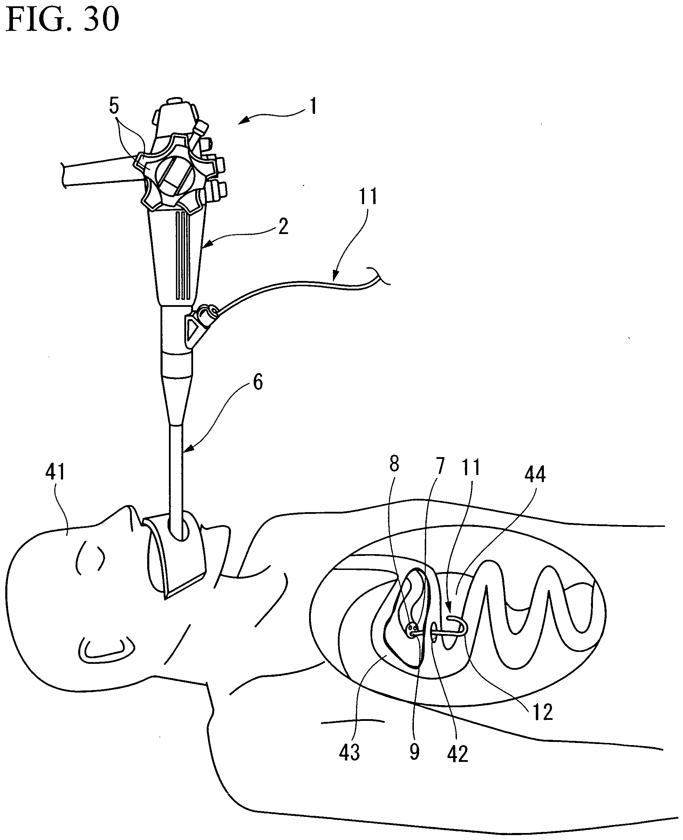

After checking that other tissues do not exist in the area around the perforation 42 (the position through which the needle 14 is passed, the puncture position, or the position at which the anchor 27 is placed), the endoscope insertion part 6 is drawn back to the inside of the stomach 43. Next, the suture instrument 11 which is passed through the channel 9 is projected. As shown in FIG 30, the tip portion of the suture instrument 11 is moved from the perforation 42 to the abdominal cavity 44. The tip portion of the suture instrument 11 is then bent to face the outer side of the stomach 43 and an area around the perforation 42 in the abdominal cavity 44.

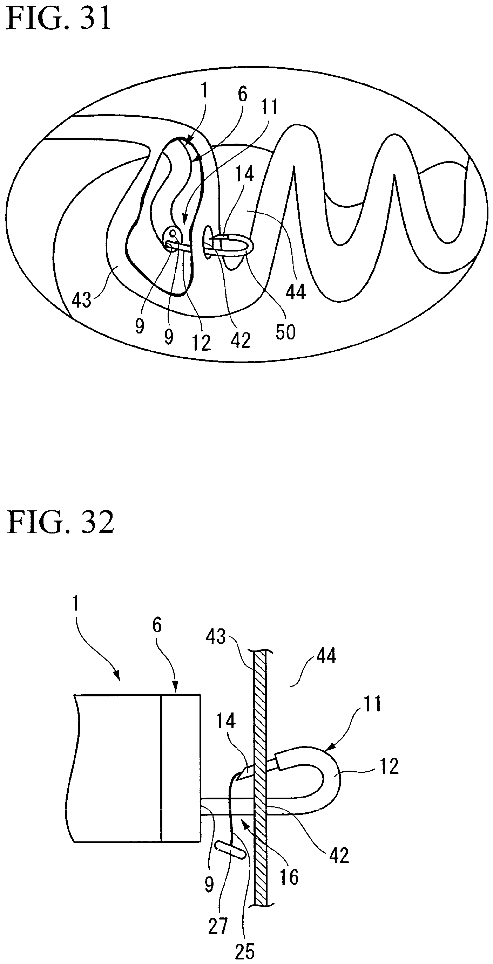

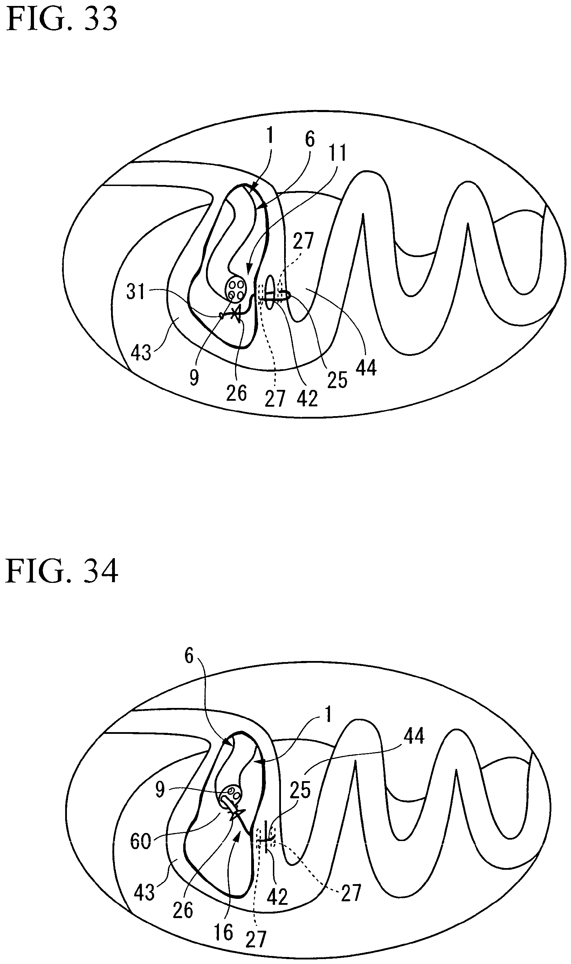

As shown in FIG. 31, the suture instrument 11 projects the needle 14 from the outer sheath 12, and the needle 14 penetrates the tissue around the perforation 42 from the abdominal cavity 44 side into the inside of the stomach 43. It is preferable that the stopper 26 be made to enter the inside of the stomach 43 when the needle 14 is projected from the outer sheath 12. As shown in FIG. 32, after the needle 14 penetrates the tissue, the first anchor 27 is pushed out and ejected to the inner side of the stomach 43. As shown in FIG. 33, after placement of two anchors 27 inside the stomach 43 so as to sandwich the perforation 42 therebetween, the suture instrument 11 is drawn back to the inside of the stomach 43, and contained in the channel 9. As shown in FIG. 34, the forceps 60 is then passed through the channel 9, and the tissue is tightened up by the suture tool 16 using the forceps 60 to suture the perforation 42. The suturing method is the same as in the second embodiment.

In this embodiment, after the inner side and the outer side of the stomach 43 are sequentially observed by the observation device 7 of the endoscope 1 to check that other tissues do not exist in an area around the perforation 42, the endoscope 1 is drawn back to the inside of the stomach 43, and the tissue is punctured with the needle 14 from the outer side of the stomach 43. Accordingly, other tissues can be easily prevented from being sutured together when suturing by using the endoscope 1.

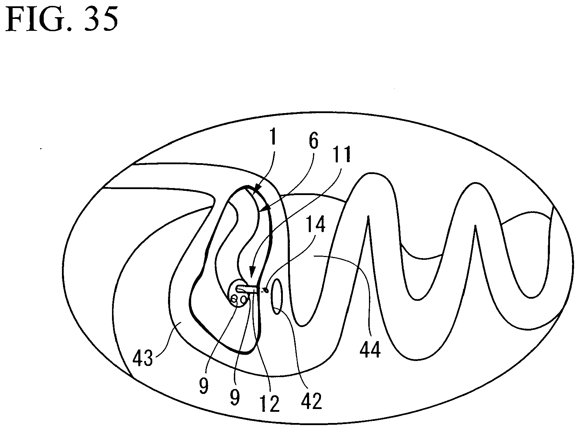

Next, modified examples of this embodiment will be explained. As shown in FIG 23, after observing the outer side of the stomach 43 by using the observation device 7 of the endoscope insertion part 6, the endoscope insertion part 6 is drawn back to the inside of the stomach 43. After that, the suture instrument 11 is projected from the endoscope insertion part 6 present in the stomach 43, and the needle 14 is thrust from the inner side to the outer side of the stomach 43, as shown in FIG 35. After placement of the anchor 27 at the outer side of the stomach 43, the suture tool 16 is tightened up to suture the perforation 42, as shown in FIGS. 11 and 12. In this case, other tissues can be easily prevented from being sutured together when suturing by using the endoscope 1.

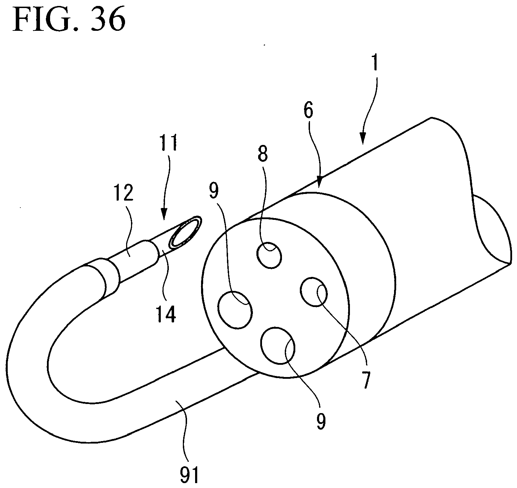

As shown in FIG. 36, a channel 91 may be provided at the periphery of the endoscope insertion part 6, and the suture instrument 11 may be passed through this channel 91. Moreover, the suture instrument 11 may be directly provided parallel to the periphery of the endoscope insertion part 6. The tip portion of the suture instrument 11 is constructed so as to be able to be independently bent.

### [Fourth Embodiment]

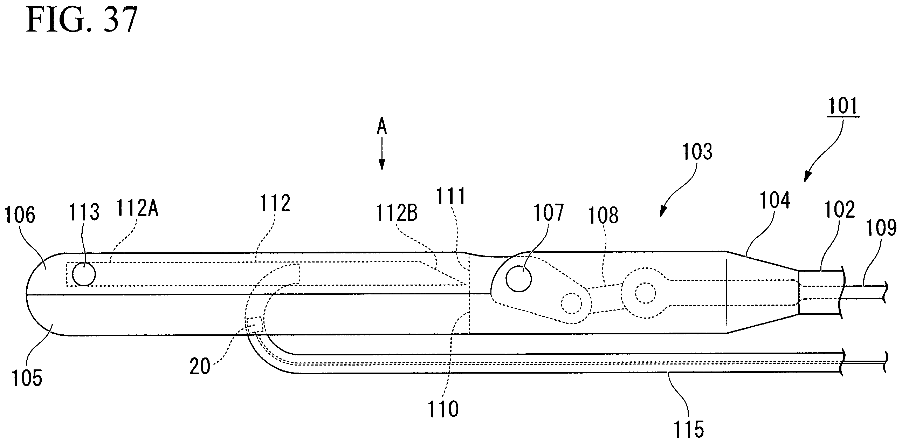

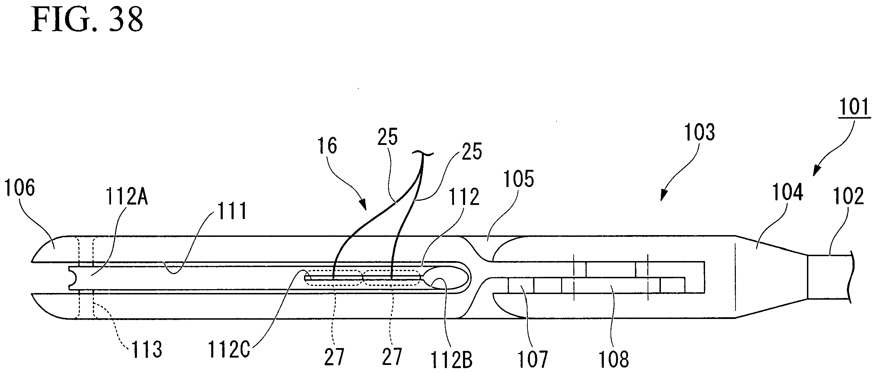

As shown in FIG. 37 and FIG 38, a treatment portion 103 that is a tip position changing mechanism is fixed at the tip which is the remote end of a sheath 102 that is a flexible base in a suture instrument 101. The treatment portion 103 has a support member 104 that is fixed to the sheath 102, and a first forceps piece 105 integrally extends from the tip of the support member 104. Moreover, a second forceps piece 106 (opening-closing member) is supported to the support member 104 so as to freely rotate by a pin 107. A portion of the second forceps piece 106 that is more to the base end side than the pin 107 is pulled into the support member 104 and connected to a link mechanism 108. The link mechanism 108 is connected to a forceps wire 109 that is passed through the sheath 102 so as to be able to freely move forward or backward. When the forceps wire 109 is moved forward or backward, the second forceps piece 106 rotates with respect to the support member 104, and so it is possible to open and close the pair of forceps pieces 105, 106. The operation unit 17 that performs the opening-closing operation (refer to FIG. 1) is fixed to the base end of the sheath 102. Note that the first forceps piece 105 may be constituted to perform the opening-closing operation.

Slits 110, 111 are formed in the pair of forceps pieces 105, 106, respectively, along the lengthwise direction thereof. A hollow needle 112 is disposed in the slit 111 of the second forceps piece 106. A tip 112A on the distal end side of the needle 112 that functions as an anchor holding instrument is disposed at the distal end portion of the second forceps piece 106, and is supported to rotate freely at the second forceps piece 106 by a pin 113. The base end side of the needle 112 is disposed at the base end side of the slit 111 and is a sharp tip 112B. In the state shown in FIG 37 and FIG. 38, the entire needle 112 is housed in the slit 111 of the second forceps piece 106 in the closed state. The needle 112 is approximately parallel with the lengthwise direction of the sheath 102, and the sharp tip 112A faces the base end side, that is, the proximal end side, of the sheath 102.

Two anchors 27 of the suture tool 16 are housed inside of the needle 112. A slit 112C extends to a predetermined length in the needle 112 from the sharp tip 112B to the tip 112A, and the suture thread 25 is drawn out from this slit 112C.

A pusher sheath 115 is connected to the side of the tip 112A of the needle 112. The pusher sheath 115 passes through the respective slits 110, 111 of the pair of forceps pieces 105, 106 and extends along the sheath 102 to be drawn out to the hand side. The pusher 20 that pushes out the anchors 27 of the suture tool 16 is passed in the pusher sheath 115 so as to be able to freely move forward or backward.

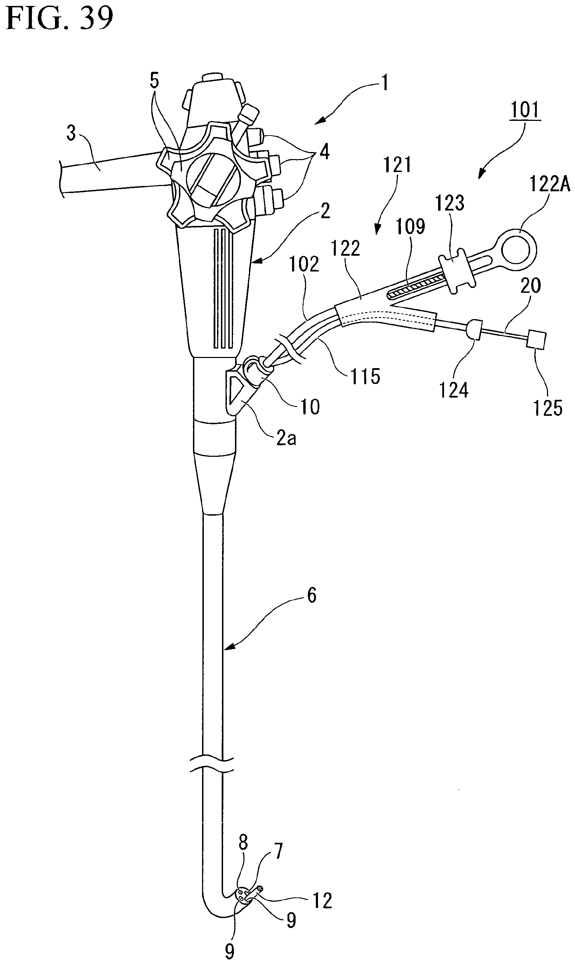

As shown in FIG. 39, a position operating device (operation unit 121) that operates the tip position changing mechanism is used by being pulled out from the endoscope 1. The operation unit 121 has an operation unit body 122 at which the base end portion of the sheath 102 is fixed. A ring 122A for placing a finger is provided on the operation unit body 122, and a slider 123 that is able to freely move forward or backward in the lengthwise direction of the sheath 102 is attached further to the distal end side than the ring 122A. The forceps wire 109 is fixed to the slider 123, and when the slider 123 is made to move forward or backward with respect to the operation unit body 122, the pair of forceps pieces 105, 106 can be opened and closed. Moreover, in the operation unit body 122, a pusher sheath 115 is passed to be able to freely move forward or backward. The pusher sheath 115 is drawn out from a position that is separated from the slider 123 by the operation unit body 122 branching into two lines. A pusher sheath knob 124 is provided so as to be easily grasped by an operator at the end portion of the pusher sheath 115 that is drawn out from the operation unit body 122. The pusher 20 is further drawn out from the pusher sheath knob 124. A pusher knob 125 is provided so as to be easily grasped by an operator at the end portion of the pusher 20.

When suturing a perforation, as shown in FIG 5, the endoscope insertion part 6 is inserted until the vicinity of the perforation 42, and performs observation of the perforation 42 from within the stomach 43. Next, as shown in FIG 23, the endoscope insertion part 6 is fed from the perforation 42 into the abdominal cavity 44, and observation of the perimeter of the perforation 42 is made from the abdominal cavity 44 side with an observation device (first observation device) 7 of the endoscope insertion part 6. After confirmation that other tissue is not in an area around the perforation 42 (the position through with the needle 112 passes, the puncture position, or the position where the anchor 27 is placed), the endoscope insertion part 6 is drawn back into the stomach 43.

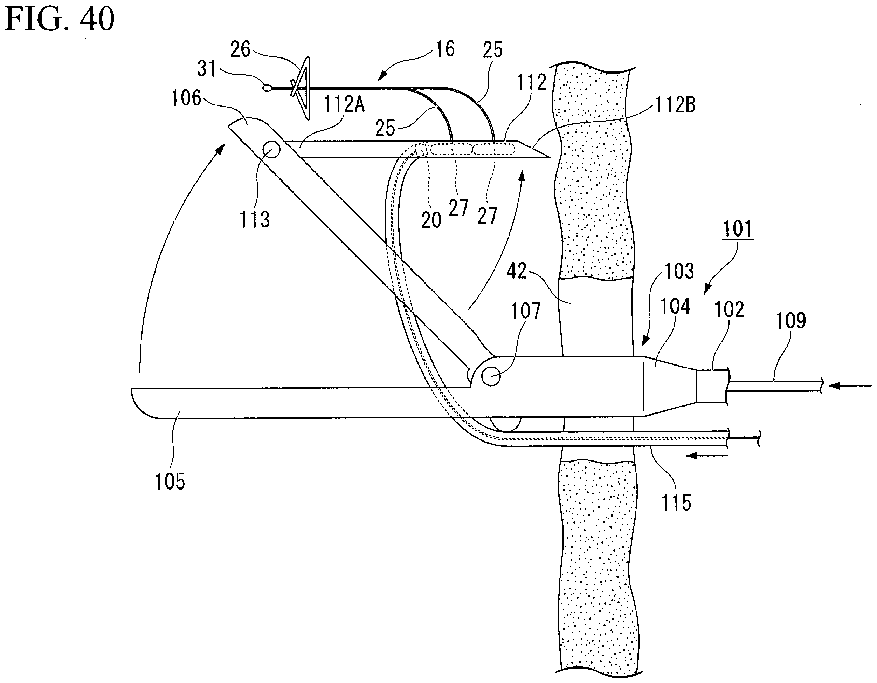

The treatment portion 103 is protruded through the perforation 42 to the outer side of the organ that is the suture object (for example, the stomach 43) with the pair of forceps pieces 105, 106 closed. By pushing in the slider 123 of the operation unit 121, the second forceps piece 106 opens with respect to the first forceps piece 105. The second forceps piece 106 that include the needle 112 moves in a direction intersecting with the axial line of the sheath 102. Moreover, by gripping the pusher sheath knob 124, the pusher sheath 115 is pushed out. As shown in FIG 40, the needle 112 that is pushed by the pusher sheath 115 rotates about the pin 113 to come out from the slit 111 of the second forceps piece 106, and the sharp tip 112B of the needle 112 faces the proximal side of the sheath 102, that is, the outer surface of the tissue around the perforation 42, at a position that is offset with respect to the axial line of the sheath 102.

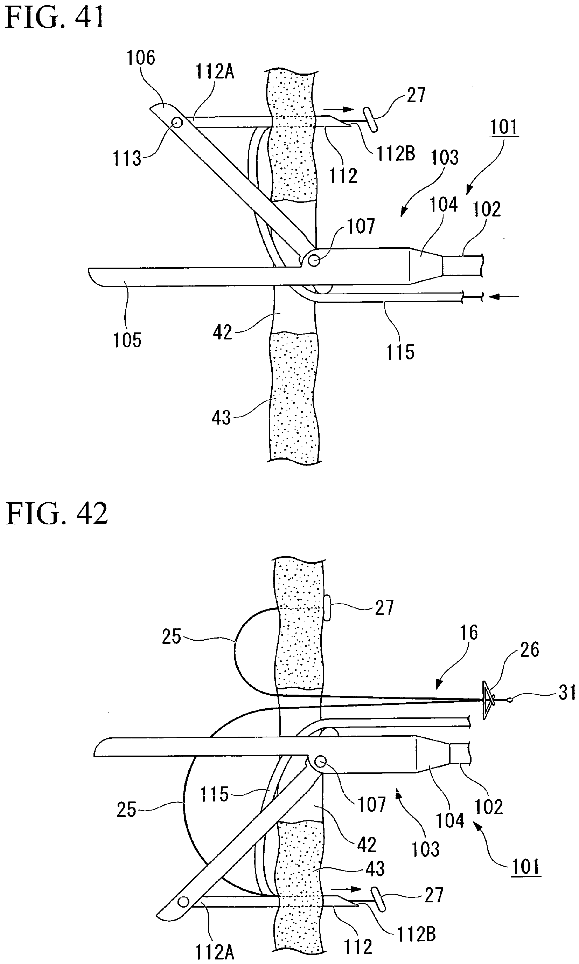

When the entire suture instrument 101 is moved backward out after aligning with the position where the sharp tip 112B of the needle 112 is to be thrust in, the needle 112 penetrates the stomach 43 from the outer side to the inner side. By grasping the pusher knob 125, as shown in FIG 41, to push in the pusher 20, a first anchor 27 is pushed out from the needle 112. Thereafter, when the entire suture instrument 101 is moved forward, the needle 112 is removed from the tissue. The suture thread 25 penetrates through the tissue, and the first anchor 27 remains in the stomach 43.

Next, the suture instrument 101 is rotated about its axial line, and the needle 112 is moved to the opposite side of the location where the suture thread 25 was passed centered about the perforation 42. Once again, when the suture instrument 101 is moved backward, the needle 112 penetrates the stomach 43 from the outer side to the inner side. As shown in FIG 42, the pusher 20 is pushed further in, and a second anchor 27 is pushed out from the needle 14. When the needle 112 is drawn out from the tissue, by grasping the pusher sheath knob 124, the pusher sheath 115 is retracted, and the needle 112 is housed in the slit 111 of the second forceps piece 106. Moreover, by pulling the slider 123, the pair of forceps pieces 105, 106 is closed.

When the suture instrument 101 is pulled out of the perforation 42 into the stomach 43, the perforation 42 is closed by tightening the suture tool 16 with the forceps 60 similarly to FIG. 28 and FIG 29.

According to this embodiment, where the needle 112 is passed from the outer side to the inner side of the tissue, it is possible to reliably pierce the needle 112. Since it is possible to place the suture tool 16 just by the opening-closing operation and the extending and retracting of the pusher sheath 115 and the pusher 20, the operation is simple. Note that the pair of forceps pieces 105, 106 can be used for holding tissue.

### [Fifth Embodiment]

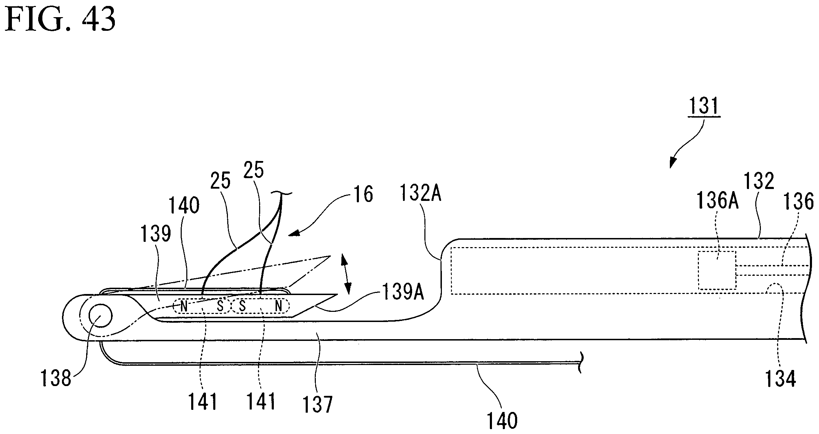

As shown in FIG 43, a suture instrument 131 has a lumen 134 in a sheath 132 that is a flexible base. An attraction surface 132A is provided so as to close the distal end of the lumen 134. An attraction instrument 136 is passed in the lumen 134 so as to move freely forward or backward. The distal end portion 136A of the attraction instrument 136 is made from a magnetic material.

At the distal end of the sheath 132, a support portion 137 extends approximately along the lengthwise direction so as to avoid the opening of the lumen 134. At the distal end of the support portion 137, a hollow needle 139 is supported so at to rotate freely by a pin 138. The needle 139 extends in an elongated manner from the end portion of the distal end side that is supported by the pin 138, and the other end portion at the base end side becomes a sharp tip 139A. In the initial state, the needle 139 is approximately parallel to the sheath 132, and the sharp tip 139A is aimed at the base end side of the sheath 132, that is, the proximal end side.

An operation wire 140 that constitutes the tip position changing mechanism is fixed to the sharp tip 139A of the needle 139. The operation wire 140 passes through the distal end of the support portion 137 to be drawn out to the hand side. When the operation wire 140 is pulled, the needle 139 rotates about the pin 138, and rises as shown by the virtual lines. The operation wire 140 has a predetermined hardness, and so by pushing in the operation wire 140, the needle 139 is housed so as to approximately follow the support portion 137, that is, to become approximately parallel to the lengthwise direction of the sheath 132. Note that by providing a groove in the support portion 137, the operation wire 140 passes along the groove, and so dropping out of the operation wire 140 is prevented.

Anchors 141 of the suture tool 16 are housed in the needle 139. In the suture tool 16, besides the two anchors 141 being manufactured from permanent magnets, it is the same as the suture tool 16. The anchors 141 are housed in the needle 139 so that the same magnetic poles face each other. In FIG 43, the S-poles face each other.

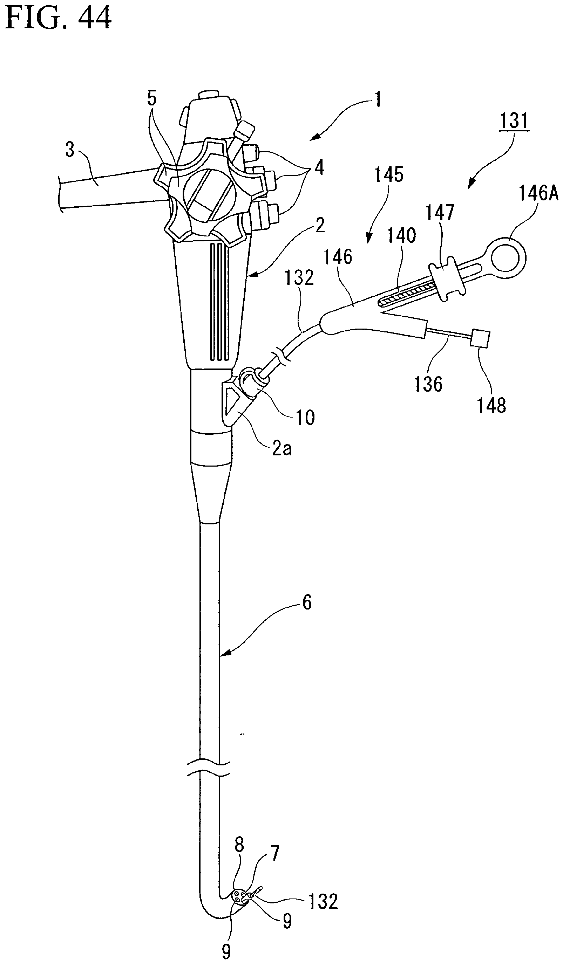

As shown in FIG. 44, an operation unit 145 that is a position operating device of the suture instrument 131 has an operation unit body 146 at which the sheath 132 is fixed. A ring 146A for placing a finger is provided on the operation unit body 146, and a slider 147 that is able to freely move forward or backward in the lengthwise direction of the sheath 132 is attached. The operation wire 140 is attached to the slider 147, and when the slider 147 is made to move forward or backward, the needle 136 can be housed and deployed. Moreover, the operation unit body 146 branches into two lines, and the attraction instrument 136 is drawn out to a position that is separated from the slider 147. A knob 148 that can be easily grasped by the operator is formed at the end portion of the attraction instrument 136.

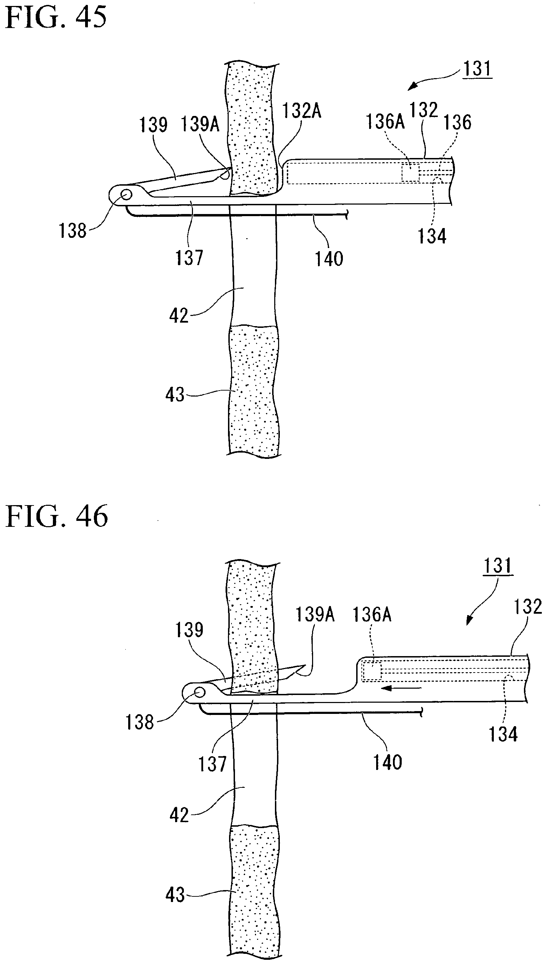

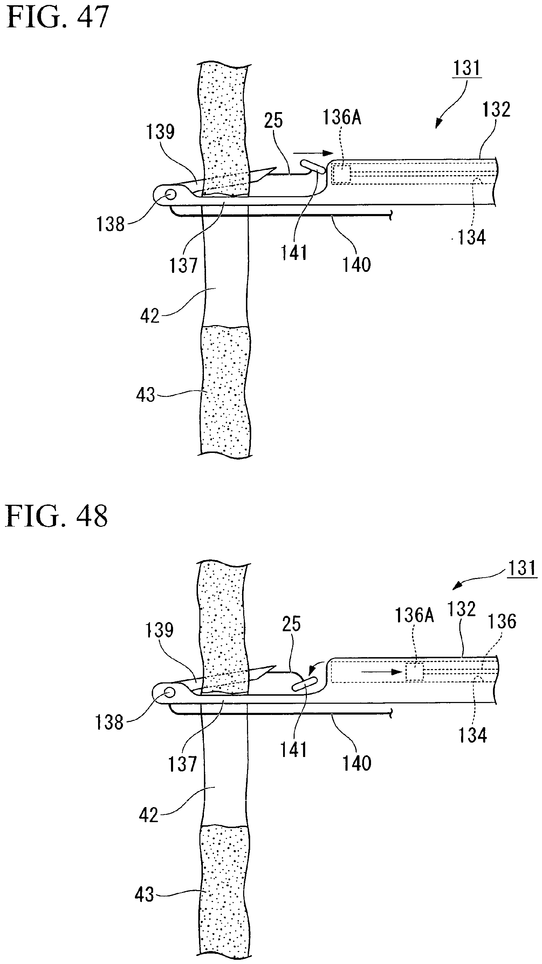

The procedure when suturing the perforation 42 with this suture tool 131 involves sequentially observing the inner side and outer side of the stomach 43 similarly to the third embodiment. When piercing the needle 139, the treatment portion 133 is protruded from the perforation 42 to the outer side. When the operation wire 140 is pulled by operating the slider 147 of the operation unit 145, the needle 139 deploys by inclining in the direction intersecting with the axial line of the sheath 132 centered on the pin 138. As shown in FIG. 45, the sharp tip 139A of the needle 139 is directed toward the tissue, that is, the proximal end side of the sheath 132. When the entire suture instrument 131 is moved backward, the needle 139 penetrates the stomach 43 from the outer side to the inner side. By grasping the knob 148 that is pulled out from the operation unit 145 to push in the attraction instrument 136, as shown in FIG 46, the attraction instrument 136 is moved toward the needle 139 from the inner side of the stomach 43. An attraction works between the distal end portion 136A of the attraction instrument 136 and the first anchor 141, whereby the anchor 141 is drawn toward the attraction surface 132A, and as shown in FIG 47, the first anchor 141 is taken out from the needle 139. After the first anchor 141 has been suctioned onto the attraction surface 132A, as shown in FIG. 48, by moving the attraction instrument 136 backward in the lumen 134, it is possible to cause the first anchor 141 to separate from the attraction surface 132A.

After removing the needle 139 from the tissue by moving the entire suture instrument 131 forward, it is rotated about its axial line, to move the needle 139 to the opposite side centered about the perforation 42. Similarly, by moving the suture instrument 131 backward, the needle 139 penetrates the stomach 43 from the outer side to the inner side. By bringing the attraction instrument 136 closer, the second anchor 141 is pulled out. By moving forward the entire suture tool 131, the needle 139 is removed from the tissue, and after the needle 139 is aligned with the support portion 137, the suture tool 131 is removed from the endoscope 1. Then, the perforation 42 is closed by tightening the suture tool 16 with the forceps 60 similarly to FIG. 28 and FIG 29.

According to this embodiment, in a suture method that passes the needle 139 from the outer side to the inner side of the tissue, it is possible to reliably pierce the needle 139. Since only a rotation operation of the needle 139 and forward-backward movement of the attraction instrument 136 are required, the operation is simple.

### [Sixth Embodiment not forming part of the present invention]

In this embodiment, a preferred suture instrument for the suture method in the third embodiment is described.

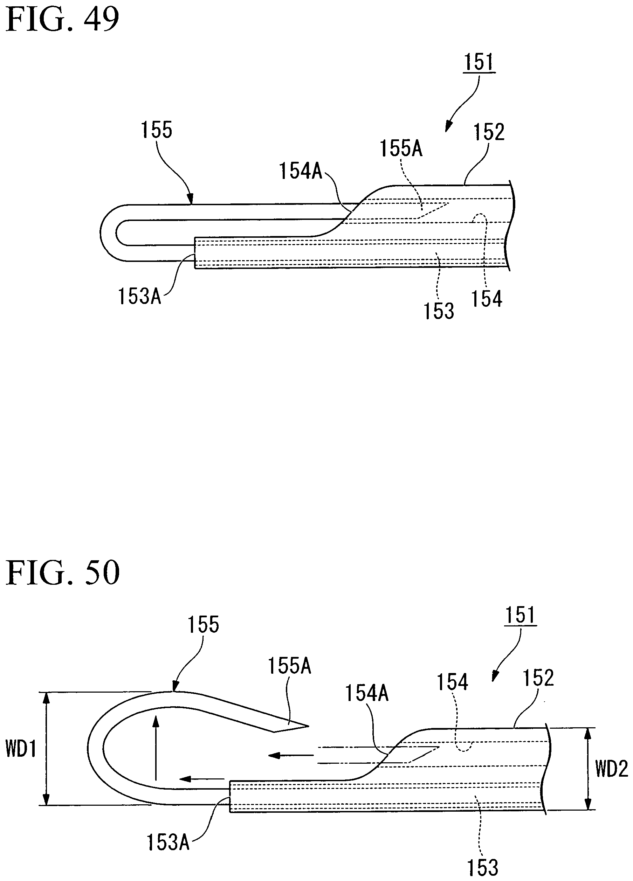

As shown in FIG 49, two lumens 153, 154 are formed approximately parallel in the lengthwise direction in the sheath 152 that is a flexible base. The first lumen 153 has an opening 153A at the distal end, and a needle 155 is passed therethrough so as to move freely forward or backward. The second lumen 154 has a distal end opening 154A which is formed further to the base end side than the distal end opening 153A deg. of the first lumen 153.

The needle 155 is hollow and long, and is made from a shape-memory alloy. A sharp tip 155A of the needle 155 protrudes from the distal end opening 153A of the first lumen 153, and after being elastically deformed in an approximate U-shape, is inserted from the distal end opening 154A to the second lumen 154 so as to be capable of being freely taken out and put in. That is, the sharp tip 155A is disposed approximately parallel with the sheath 152 and disposed facing the proximal end of the sheath 152.

The needle 155 is an anchor housing tool in which two anchors 27 (not illustrated in FIG. 49) of the suture tool 16 are housed at the sharp end portion 155A. The anchors 27 can be pushed out by the pusher 20 that is passed through the needle 155. Note that the needle 155 is curved in a loop shape and stores a shape so that the sharp tip portion 155A faces the base end side. As shown in FIG 50, when pulled out from the second lumen 154, the curved needle 155 deploys in the direction of intersecting the axial line of the sheath 152. An external width WD1 of the needle 155 at this time is greater than an external width WD2 of the sheath 152. Note that in this embodiment, the curved portion of the needle 155 is a tip position changing mechanism, and the end portion at the hand side of the needle 155 is a position operation device.

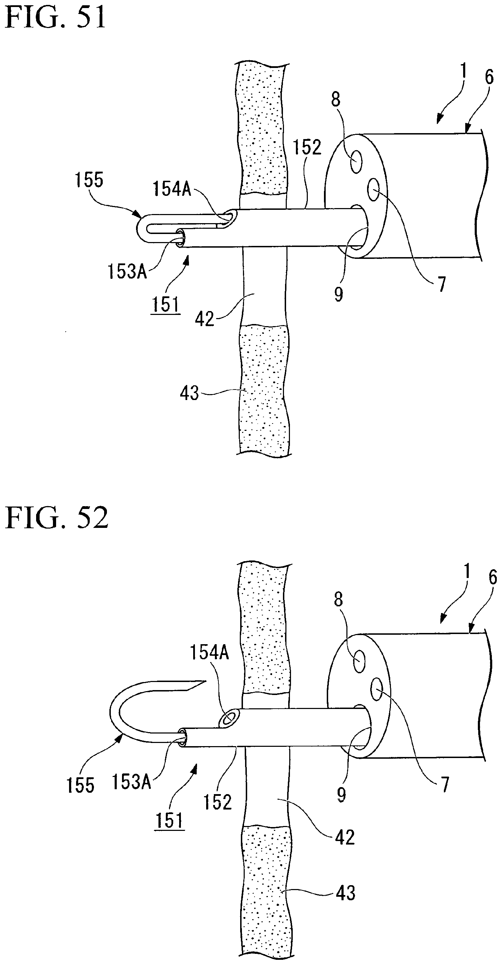

The procedure when suturing the perforation 42 with this suture tool 151 involves sequentially observing the inner side and outer side of the stomach 43 similarly to the third embodiment. As shown in FIG 51, when piercing the needle 155, the suture instrument 151 is protruded to the outer side from the perforation 42 until the formation position of the distal end opening 154A of the second lumen 154. When the needle 155 is pushed with respect to the sheath 152, the sharp tip 155A of the needle 155 comes out from the second lumen 154. As shown in FIG. 52, the needle 155 deploys due to the self-restoring force, and the sharp tip 155A moves from a position that is approximately parallel to the sheath 152 to a position that is offset from the axial line of the sheath 152 to be directed at tissue around the perforation 42. By moving the entire suture instrument 151 backward, the needle 155 penetrates the stomach 43 from the outer side to the inner side. When one anchor 27 is pushed out and released by the pusher 20, the suture instrument 151 is moved forward to remove the needle 155 from the tissue, and then similarly passed through tissue on the opposite side of the perforation 42. When a second anchor 27 is pushed out and released by the pusher 20, the needle 155 is removed from the tissue and then the suture instrument 151 is withdrawn. Then, the perforation 42 is closed by tightening the suture tool 16 with the forceps 60 similarly to FIG 28 and FIG 29.

According to this embodiment, the needle 155 that is made to have a shape memory is curved to be housed in the second lumen 154, whereby the suture instrument 151 can be readily passed through the endoscope 1 and the perforation 42. When deploying the needle 155, it only needs to be moved forward, so the operation is easy.

Note that instead of the anchors 27 and the pusher 20, it is possible to use the anchors 141 and the attraction instrument 136 of the fourth embodiment.

### [Seventh Embodiment]

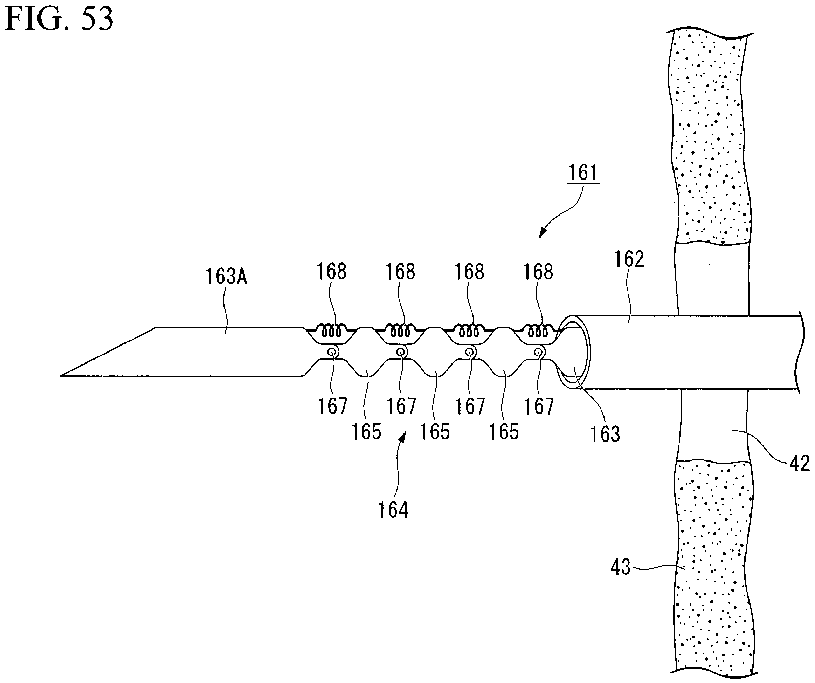

As shown in FIG 53, in a suture instrument 161, a hollow needle 163 that is an anchor holding instrument is passed through a sheath 162 that is a flexible base. The needle 163 has flexibility, and after a curving portion 164 that is a deflection mechanism is provided at a portion protruding from the sheath 162, a sharp tip 163A that is to be passed through tissue is provided. The curving portion 164 has a constitution in which a plurality of curving pieces 165 is connected in the lengthwise direction. Each curving piece 165 consists of a hollow member, and each base end portion and distal end portion thereof is attached by a pin 167 to another adjacent curving piece 165 so as to rotate freely. The pins 167 that mutually link each curving piece 165 are all disposed approximately in parallel. Moreover, a curving control member 168 that consists of a shape-memory alloy is extended between each curving piece 165. Each curving control member 168 is disposed at the same position in the circumferential direction viewed from the center line of the needle 163, and each is wound in a coil shape in the initial state. That is, in the initial state, the needle 163 is disposed approximately parallel with the axial line of the sheath 162, and the sharp tip 163A faces the remote end direction of the sheath 162. The curving control member 168 can be restored to an approximately straight shape by passing electricity, heat generation, or body temperature. For this reason, the position operation device of this suture instrument 161 is the curving control member 168 itself in the case of restoring to the original shape by a device that passes electricity through or generates heat in the curving control member 168, or by body heat.

The procedure when suturing the perforation 42 with this suture instrument 161 involves sequentially observing the inner side and outer side of the stomach 43 similarly to the third embodiment.

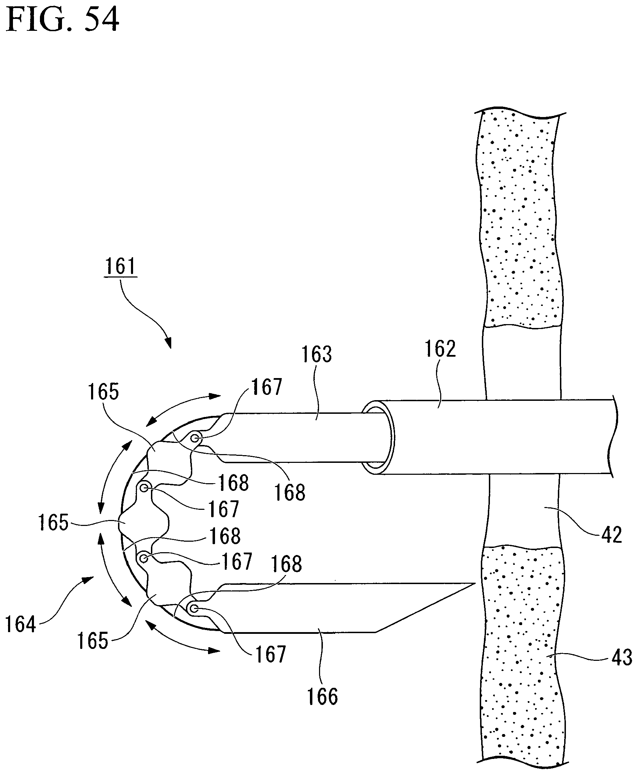

In the state of contracting the curving control members 168 into a coil shape, that is, in the state of the needle 163 being disposed so as to be approximately parallel to the sheath 162, the needle 163 is passed through the perforation 42. When the curving portion 164 is projected to the outer side from the perforation 42, electrical current is supplied to the curving control members 168 or the curving control members 168 are heated. When each curving control members 168 is extended by reverting to the original shape, as shown in FIG 54, the adjacent curving pieces 165 and the needle 163 are pushed in a certain direction. As a result, the curving portion 164 curves substantially in a U-shape, and the sharp tip 163A of the needle 163 is directed from the remote end direction of the sheath 162 to the proximal end side, that is, the tissue.

By extending and retracting movement of the needle 163, the needle 163 penetrates the stomach 43 from the outer side to the inner side, and the first anchor 27 is pushed out from the needle 163 by the pusher 20. When the suture thread 25 is passed through at two locations that sandwich the perforation 42 and the anchors 27 are pushed out and released into the inner side of the stomach 43, the suture instrument 161 is withdrawn from the endoscope 1. Then, the perforation 42 is closed by tightening the suture tool 16 with the forceps 60 similarly to FIG 28 and FIG. 29.

According to this embodiment, by using a shape-memory alloy in a portion of the curving portion 164 of the needle 163 to perform curving in the body, when withdrawing the suture instrument 161, it is possible to perform handling with the needle 163 in a straight line, and so the operation is easy.
Here, the curving control members 168 that consist of a shape-memory metal are not limited to a shape that winds a coil in a coil shape. Also, the curving control members 168 may be a plate-shaped member that curves by being supplied electrical current or the like. It is possible to use the plate-shaped curving control members 168 together with the curving pieces 165 or in place of the curving pieces 165.

### [Eighth Embodiment not forming part of the present invention]

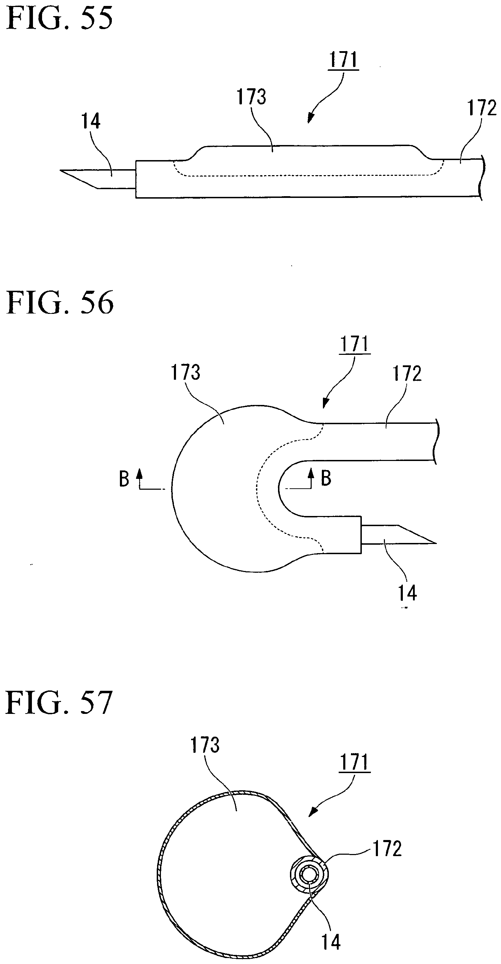

In this embodiment, a preferred suture instrument for the suture method in the third embodiment is described.
As shown in FIG. 55, in a suture instrument 171 a needle 14 is passed in a sheath 172 that is a flexible base so as to be able to move backward or forward. A balloon 173 that is a deflection mechanism is integrally formed at the distal end portion of the sheath 172. The balloon 173 is provided at only a portion of the outer circumferential surface, and does not continue over the entire circumference. Therefore, it is offset from the center line of the needle 14 that passes through the center of the sheath 172.

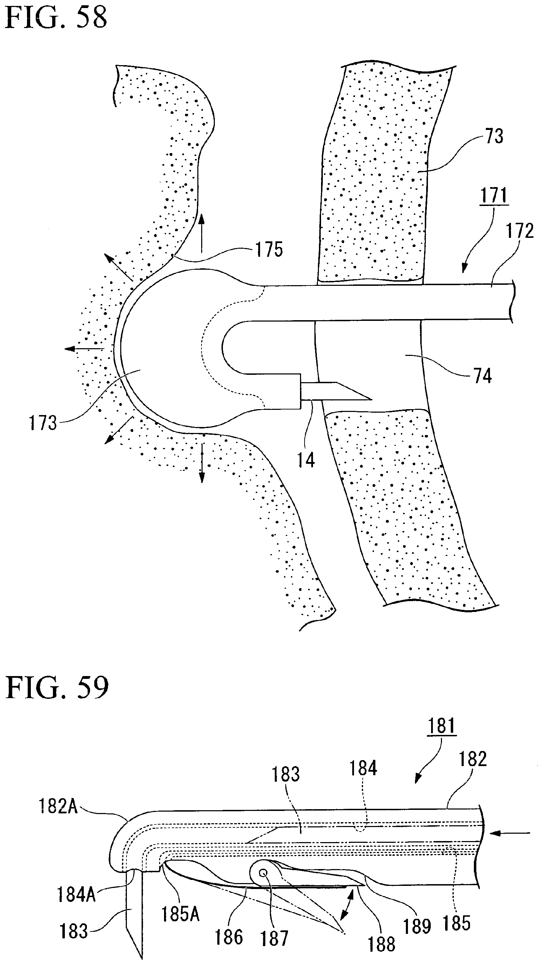

Accordingly, in the state of the sharp tip of the needle 14 being protruded to the outer side from the perforation 42, a fluid supply device (position operation device) is attached to the end portion of a lumen that is not illustrated, and so when a fluid is supplied, the balloon 173 swells as shown in FIG. 56 and FIG. 57, and the sheath 172 curves by being pressed by the balloon 173. Since the balloon 173 is disposed so that the sheath 172 curves in an approximate U-shape, the sharp tip of the needle 14 is directed from the distal direction of the sheath 172 toward the proximal end side, that is toward the tissue, and so it is possible to pierce through the tissue from the outer side to the inner side. Here, as shown in FIG. 58, an outside organ 175 that is in the area of the tissue that is the suture object is excluded by the swelling of the balloon 173.

In this embodiment, since it is possible to curve the needle 14 in an approximate U-shape by swelling the balloon 173, it becomes possible to pierce through the tissue from the outer side to the inner side with a simple operation. Moreover, since the balloon 173 excludes the outside organ 175, it is possible to simply perform suturing even at locations that are in the proximity of the outside organ 175.

### [Ninth Embodiment not forming part of the present invention]

In this embodiment, a preferred suture instrument for the suture method in the third embodiment is described.
As shown in FIG 59, in a suture instrument 181 a needle 183 is passed in a sheath 182 that is a flexible base so as to be able to move backward or forward. Two lumens 184, 185 are formed approximately parallel in the sheath 182. Since a curving portion 182A that functions as a deflection mechanism is formed at the distal end that is a remote end of the sheath 182, a first lumen 184 forms an opening 184A at the side portion by being curved approximately 90 degrees. A hollow needle 183 that is an anchor holding instrument is passed through the first lumen 184 so as to freely move forward or backward. It is preferable to manufacture the needle 183 with a super-resilient alloy, so that even when passing through a lumen that is positioned at the curving portion 182A, a permanent deformation is not produced.

The second lumen 185 is curved by approximately 90 degrees by the curving portion 182A, and at further to the base end side than the first lumen 185, an opening 185A is formed at the same side portion in the circumferential direction. An operation wire 186 is passed through the second lumen 185 so as to be able to freely move forward or backward. The operation wire 186 is fixed to the distal end portion of an erecting hook 188 that is supported in the sheath 182 so as to freely rotate by a pin 187 further to the base end side than the opening 185A. The erecting hook 188 can be housed in a recessed portion 189 that is formed in the sheath 182 so that the distal end portion thereof faces the hand side. Thereby, when the operation wire 186 is pulled, the erecting hook 188 deploys to the outer side as shown by the virtual lines.

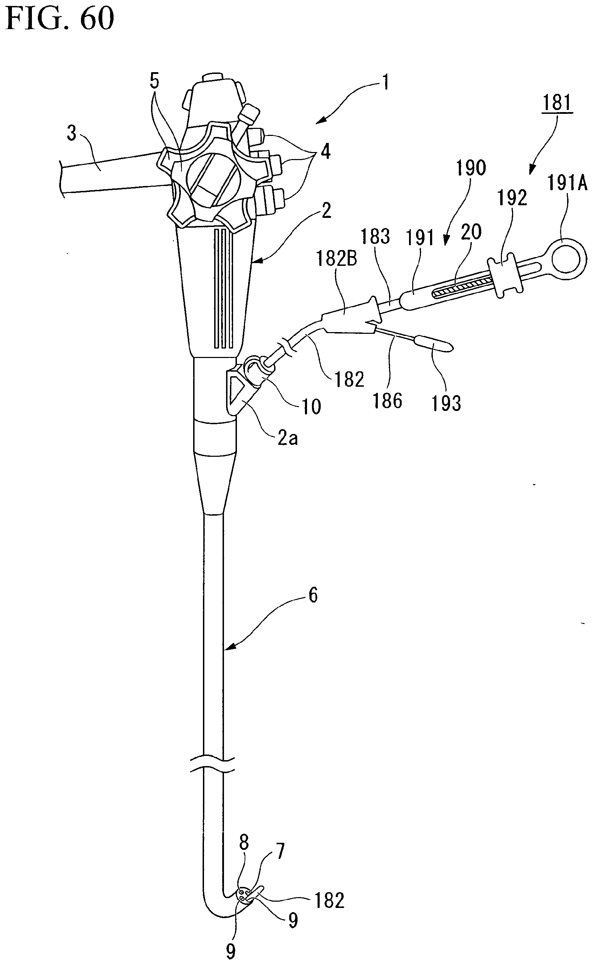

As shown in FIG. 60, an operation unit 190 that is the position operation device of the suture instrument 181 is constituted to include a base end portion 182B of the sheath 182. When the base end portion 182B is grasped and moved forward or backward, it is possible to move the entire suture instrument 181 forward and backward. The needle 183 and the operation wire 186 are separately pulled out from the base end portion 182B. An operation unit body 191 is fixed to the needle 183. A ring 191 A for placing a finger is provided on the operation unit body 191, and a slider 192 that is able to freely move forward and backward in the lengthwise direction is attached. A pusher 20 is fixed to the slider 192. A knob 193 that can be easily grasped by the operator is formed at the end portion of the operation wire 182.

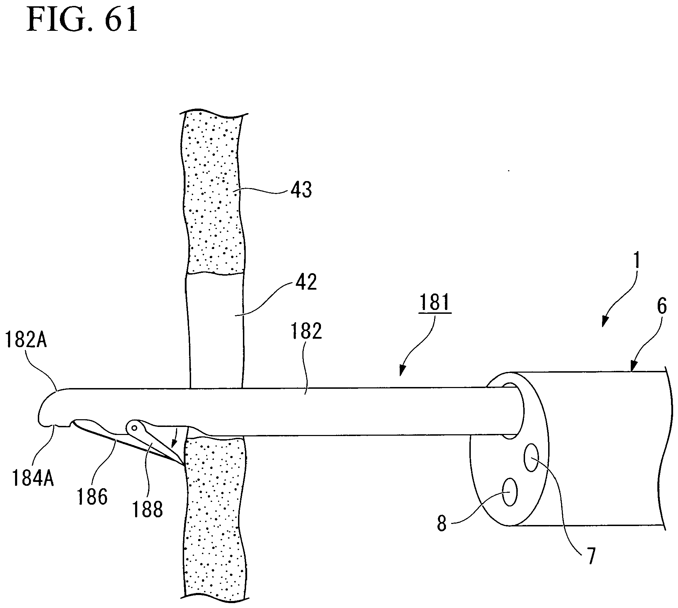

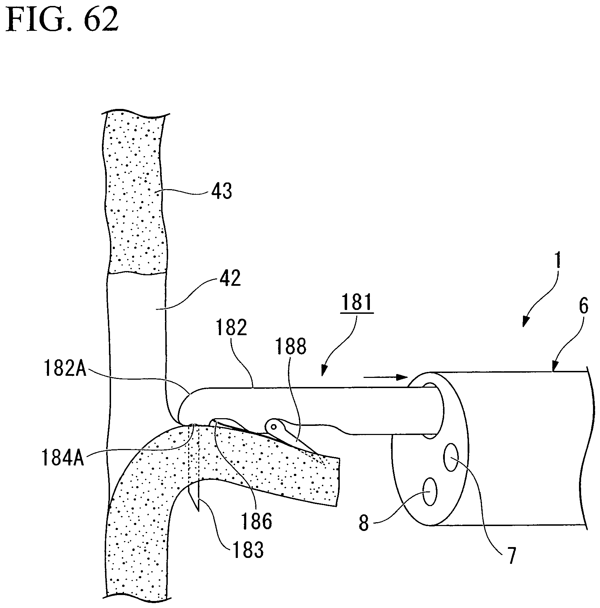

As shown in FIG 61, when suturing the perforation 42, in the state of the needle 183 being disposed at the base end side of the first lumen 184, that is, the sharp tip of the needle 183 being housed approximately parallel in the sheath 182, it is protruded to the outer side of the perforation 42. When the erecting hook 188 is sent to the outer side through the perforation 42, by pulling the operation wire 186 the erecting hook 188 is deployed. When the tissue around the perforation 42 is caught on the distal end of the erecting hook 188 and the entire suture instrument 181 is moved backward, the tissue that is caught on the erecting hook 188 is drawn in toward the endoscope 1. As shown in FIG 62, since orientation of the opening 184A of the first lumen 184 and the tissue that is drawn in intersect, when the needle 183 is moved forward, the sharp tip thereof is deflected approximately 90 degrees from the remote direction of the sheath 182 to the direction heading toward the proximal end, and so it is possible to pierce through the tissue that is caught on the erecting hook 188.

When the needle 183 is pierced through and the first anchor 27 has been pushed out and released, after the needle 183 is removed the entire suture instrument 181 is moved forward. The engagement between the erecting hook 188 and the tissue is thus released. The needle 183 is then pieced through the tissue on the opposite side viewed from the perforation 42 while similarly drawing in the tissue with the erecting hook 188 and the second anchor 27 is pushed out. After the suture instrument 181 has been removed from the endoscope 1, the perforation 42 is closed by tightening the suture tool 16 with the forceps 60 similarly to FIG 28 and FIG 29.

According to this embodiment, since the needle 183 is pierced through the tissue after it has been drawn in with the erecting hook 188, it is possible to place the suture tool 16 with a simple operation. Moreover, by drawing in the tissue with the erecting hook 188, it is possible to observe with the endoscope 1 the position and piercing process when thrusting the needle 183 without curved deformation of the needle 183 by approximately 180 degrees as in other embodiments. Instead of hooking the tissue on the erecting hook 188, the tissue may also be sandwiched between the erecting hook 188 and the sheath 182.

Here, since the curving portion 182A at the distal end of the sheath 182 may be an angle that allows the needle 183 to pierce through the tissue in cooperation with the erecting hook 188, and may be greater than 0 degrees and equal to or less than 90 degrees, compared to the case of curving by approximately 180 degrees, the operation of moving the pusher 20 forward and backward and pushing out of the anchors 27 can be reasonably performed with a light force.

### [Tenth embodiment not forming part of the present invention]

In this embodiment, a preferred suture instrument for the suture method in the third embodiment is described.

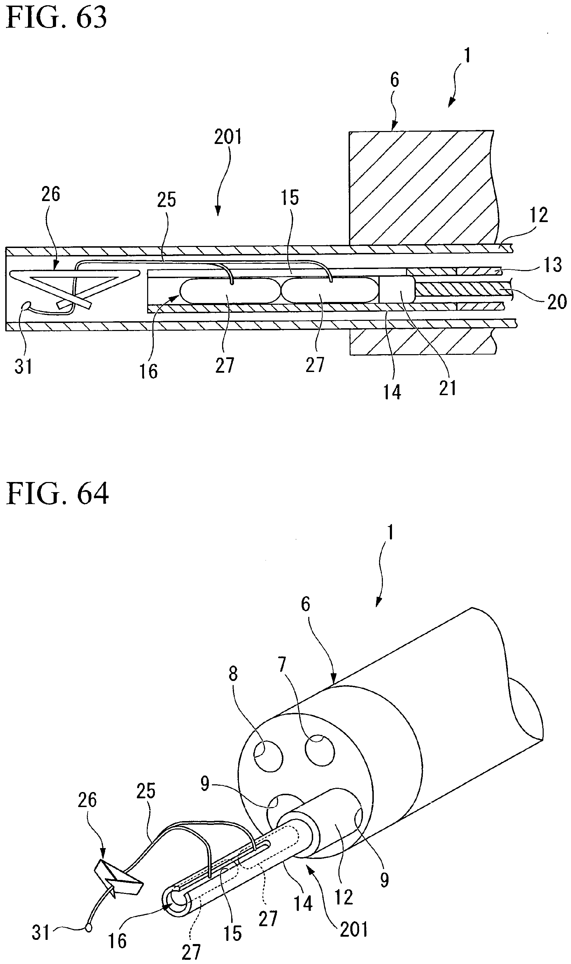

As shown in FIG 63 and FIG. 64, in a suture instrument 201, an inner sheath 13 that functions as an anchor holding instrument that is a flexible base is passed inside of a flexible outer sheath 12 so as to freely move forward and backward. In the sinner sheath 13, a slit 15 that opens at the distal end is provided along the lengthwise direction of the side portion. In the interior of the inner sheath 13, two anchors 27 of the suture tool 16 are housed in line. The pusher 20 is made to abut the base end side anchor 27. Note that as the operation unit of this suture instrument 210, it is possible to use the same one as the operation unit 17 shown in FIG. 1.

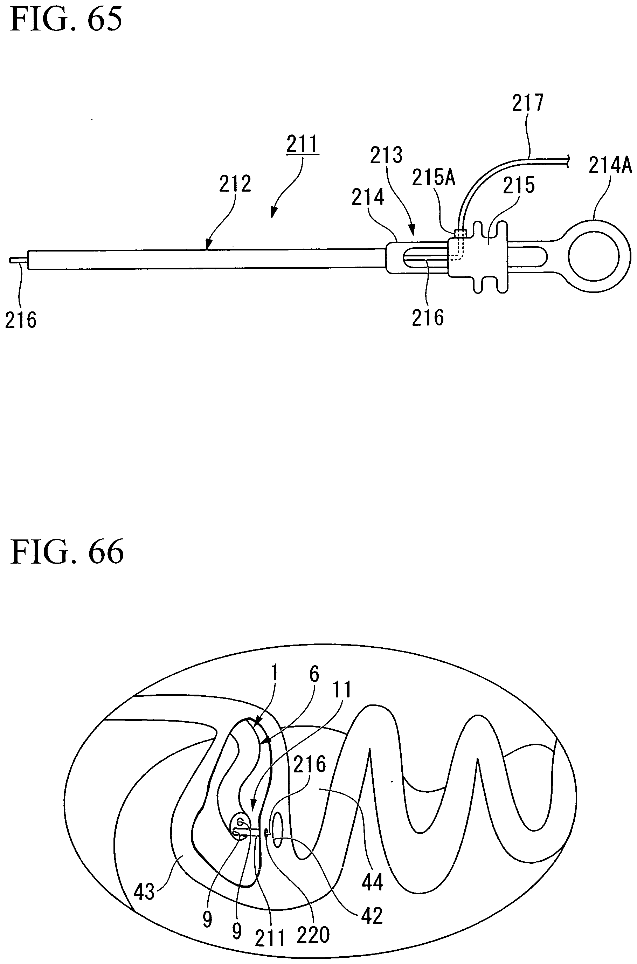

In this suture instrument 201, a treatment instrument such as a high-frequency knife and the like is used for passing the suture thread 25 of the suture tool 16. FIG. 65 shows a high-frequency knife that is an example of a treatment instrument. The high-frequency knife 211 has a long sheath 212 having flexibility, and an operation unit 213 is provided at the base end of the sheath 212. A slider 215 that is able to move forward and backward with respect to the operation unit body 214 and a ring 214A for placing a finger are provided in the operation unit 23. The end portion of a long electrode 216 is fixed to the slider 215. The electrode 216 is electrically connected to an electrode cable 217 via a terminal 215A that is provided in the slider 215. The electrode cable 217 is connected to a high-frequency power supply not illustrated. The electrode 216 passes through the sheath 212 so as to be capable of freely moving forward and backward, and extends to the distal end of the sheath 212. When the slider 215 is moved backward, the electrode 216 is housed in the sheath 212. When the slider 215 is moved forward, the distal end portion of the electrode 216 protrudes from the sheath 212.

As shown in FIG 66, when suturing the perforation 42, the high-frequency knife 211 is passed through the channel 9 of the endoscope 1, and a small incision hole 220 is formed from the inner side to the outer side in tissue around the perforation 42. Specifically, when the predetermined position where the small incision hole is to be formed is confirmed, the slider 215 is moved forward to cause the electrode 216 to protrude from the sheath 212. By supplying a high-frequency current from the high-frequency power supply, the distal end of the electrode 216 is pressed against the predetermined position to form the small incision hole 220. When the small incision hole 220 is formed at two locations sandwiching the perforation 42, the supply of high-frequency current is stopped. After housing the electrode 216 in the sheath 212, the high-frequency knife 211 is removed from the endoscope.

Next, the suture thread 25 is passed through the small incision hole 220 and then anchors 27 are placed one at a time. The suture instrument 201 is passed through the endoscope 1, and the inner sheath 13 is protruded from the outer sheath 12. At this time, the inner sheath 13 abuts the stopper 26 with a wide area, and pushes out the stopper 26 from the outer sheath 12 into the stomach 43.

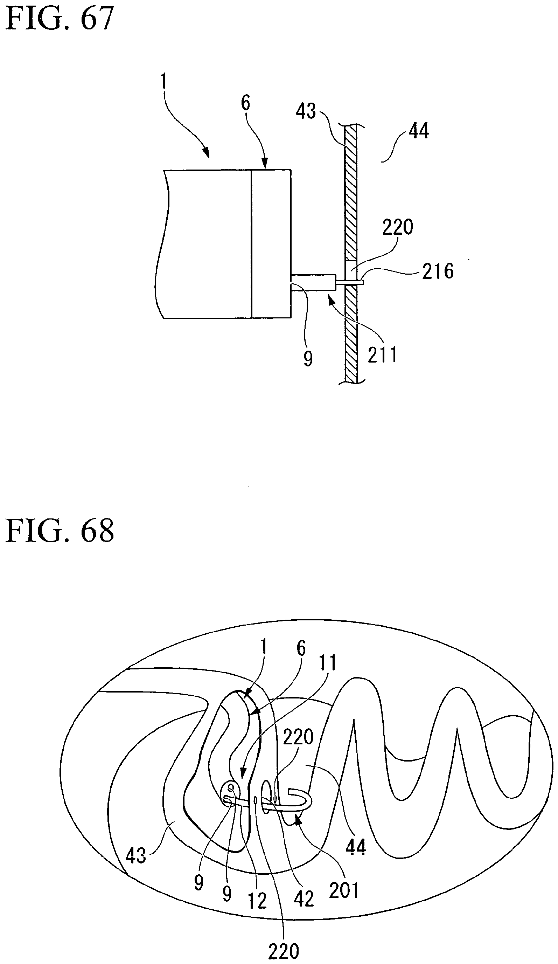

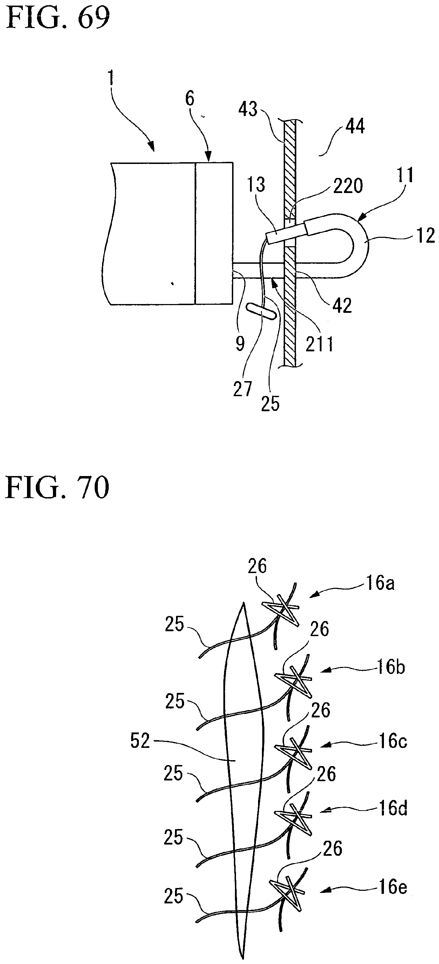

As shown in FIG. 68, the outer sheath 12 of the suture instrument 201 is protruded into the abdominal cavity 44 through the perforation 42. The distal end portion of the outer sheath 12 is curved, and the outer sheath 13 is directed from a direction toward the remote end of the suture instrument 201 to a direction toward the proximal end, that is, to the tissue. Moreover, by moving the inner sheath 12 forward, it passes through the small incision hole 220 from the outer side to the inner side. Since the inner sheath 13 protrudes from the abdominal cavity 44 into the stomach 43, when the pusher 20 is moved forward as shown in FIG. 69, the first anchor 27 is pushed out and released in the stomach 43. Then, the inner sheath 13 is drawn out from the small incision hole 220. The suture thread 25 passes through the small incision hole 220. Moreover, the inner sheath 13 is similarly passed through the small incision hole 220 on the opposite side. When the second anchor is released into the inside of the stomach 43, the suture instrument 201 is pulled back, causing the suture thread 25 to pass through the small incision hole 220. The suture instrument 201 is pulled back into the stomach 43, and when the suture tool 16 is tightened similarly to the previous embodiments, the perforation 42 is sutured.

Since this suture instrument 201 is of a constitution that does not have a needle, a wide contact surface with the stopper 26 is provided, and so the stopper 26 can easily and reliably be pushed out from the outer sheath 12.

This invention can be widely applied without being limited to the above-mentioned embodiments.

For example, in each suture instrument from the fourth embodiment to the eight embodiment, it is possible to use an anchor holding instrument such as shown in the ninth embodiment in place of a needle, with the anchors being released after passing the anchor holding instrument through a small incision hole that is formed with another treatment tool.

For example, the endoscope 1 may be inserted from the anus into the large intestine which is an example of a hollow organ. In this case, a perforation formed in the large intestine is sutured. Although the perforation 42 is described as being already formed, the procedure of the above-mentioned embodiment may be carried out after forming the perforation 42 by using the endoscope 1. In this case, the endoscope 1 is inserted from a natural opening into the inside of the stomach 43, and a predetermined incision portion is checked by the observation device 7 provided at the tip of the endoscope insertion part 6. After that, the predetermined incision portion is incised after passing a high-frequency knife or the like through the channel 9 of the endoscope 1 to form the perforation 42.

When the stomach 43 is widely incised and the perforation 42 is sutured by using at least three suture tools 16, suture tools 16 plurally lined up are preferably sequentially tightened up from one end thereof. In an example shown in FIG. 70, a suture tool 16a, a suture tool 16b, a suture tool 16c, a suture tool 16d, and a suture tool 16e are tightened up in this order, for example. Since the perforation 42 is sutured from one end thereof, and the size of the perforation 42 can be gradually diminished, suturing can be easily carried out. Alternatively, the suture tool 16 at the center of the suture tools 16 lined up may be tightened up first, followed by tightening the suture tools 16 at the center positions between the suture tool 16 tightened up at the center position and the suture tools 16 at the ends thereof. In the example shown in FIG 70, the suture tool 16c is tightened up first, the suture tool 16b and the suture tool 16d are then tightened up, and the suture tool 16a and the suture tool 16e are finally tightened up. Since the center position of the opening is always sutured, the degree of slippage of suture positions can be diminished.

### [INDUSTRIAL APPLICABILITY]

The suture instrument according to this invention may be preferably utilized for medical applications.

## Claims

1. A suture instrument (101, 131, 151) adapted to be inserted into a body and to eject elongated anchors (27, 141) to which a suture thread (25) is connected, comprising:
a base (13, 102, 152) that extends from a proximal end on a hand side to a remote end that is introduced to tissue, the base (13, 102, 152) being capable of being inserted into a perforation and having flexibility;
an anchor holding instrument (112, 139, 155) that is capable of housing a plurality of the anchors (27, 141) inside,
wherein the anchor holding instrument (112, 139, 155) is disposed and housed in the remote end of the base (13, 102, 152) along an axis of the base (13, 102, 152) such that the distal end of the anchor holding instrument (112, 139, 155) is approximately pointed in a direction of a proximal end of the base (13, 102, 152);
a tip position changing mechanism (103, 140) adapted to move the distal end of the anchor holding instrument (112, 139, 155) in a radial direction intersecting the axial line of the base (13, 102, 152) from a position where the anchor holding instrument (112, 139, 155) is housed in the base (13, 102, 152) to a position where the distal end of the anchor holding instrument (112, 139, 155) is apart from a distal end of the base (13, 102, 152) and is approximately pointed in the direction of the proximal end of the base (13, 102, 152); and
a position operation device (17, 121, 145, 190) that operates the tip position changing mechanism (103, 140) at the hand side and operates a position of the distal end of the anchor holding instrument (112, 139, 155),
**characterized in that** a distal end of the anchor holding instrument (112, 139, 155) is adapted to be inserted into the tissue and to eject the anchors (27, 141).

2. The suture instrument (101, 131, 151) according to claim 1, wherein the tip position changing mechanism (103, 140) changes the position by causing the anchor holding instrument (112, 139, 155) to move to a position that is offset from an axial line in a lengthwise direction of the base (13, 102, 152).

3. The suture instrument (101, 131, 151) according to claim 1, wherein
the tip position changing mechanism (103, 140) changes the position by inclining the anchor holding instrument (112, 139, 155) with respect to an axial line in a lengthwise direction of the base (13, 102, 152).

4. The suture instrument (101, 131, 151) according to any one of claims 1 to 3, wherein the anchor holding instrument (112, 139, 155) is a needle (112, 139, 155) that has at the distal end a sharp tip.

5. The suture instrument (101, 131, 151) according to any one of claims 1 to 3, wherein the distal end of the anchor holding instrument (112, 139, 155) consists of a hollow needle (112, 139, 155), and the anchors (27, 141) are disposed in the needle (112, 139, 155).

6. The suture instrument (101, 131, 151) according to any one of claims 1 to 5, further provided with an attraction instrument (136) that attracts the anchors (27, 141) with a magnetic force, wherein
the base has a lumen (134);
the attraction instrument (136) is disposed along the lumen (134) of the base (13, 102, 152) so as to freely advance and retract, and the anchors (27, 141);
and the attraction instrument (136) are both made from a magnetic body, and one of them is a magnet.

7. A suture instrument (161) adapted to be inserted into a body and to eject elongated anchors (27, 141) to which a suture thread (25) is connected, comprising:
a base (162) that extends from a proximal end on a hand side to a remote end adapted to be introduced to tissue, the base (162) being capable of being inserted into a perforation and having flexibility; and
an anchor holding instrument (163) that is disposed at the remote end of the base (13, 102, 152, 162), and is capable of housing a plurality of the anchors (27, 141) inside,
a distal end of the anchor holding instrument (163) is adapted to be inserted into the tissue and to eject the anchors (27, 141), wherein the distal end of the anchor holding instrument (163) is disposed and housed in the remote end side of the base (162), the anchor holding instrument (163) being pointed in the remote end of the base (162) along an axis of the base (162);
the suture instrument (161) further comprising
a deflection mechanism (164) that is provided for deflecting a position of the distal end of the anchor holding instrument (163) and causes the distal end of the anchor holding instrument (163) to deflect from the remote end of the base (162) toward a direction that faces the proximal end; and,
wherein
the anchor holding instrument (163) is capable of penetrating tissue in a state that the position of the distal end of the anchor holding instrument (163) which is protruded from the base (162) is deflected in a direction from the distal end of the base (162) toward a proximal end of the base (162) by the deflection mechanism (164) and is capable of ejecting the anchors (27, 141) at a proximal side of the tissue,
**characterized in that** the deflection mechanism (164) includes a plurality of curving pieces (165), a plurality of pins (167) connecting the curving pieces (165) to adjacent curving pieces so as to rotate freely about the pins (167), and a curving control member (168) made of shape-memory material and disposed in a line along the deflection mechanism (164) at the side of the curving control member (168), the deflection mechanism (164) is configured to deflect the position of the distal end of the anchor holding instrument (163) by the curving control member (168) being restored to an original shape.

8. The suture instrument (161) according to claim 7, wherein the deflection mechanism (164) changes the position by inclining the anchor holding instrument (163) with respect to an axial line in the lengthwise direction of the base (162).

9. The suture instrument (161) according to claim 7 or 8, wherein the anchor holding instrument (163) is a needle that has at the distal end a sharp tip.

10. The suture instrument (161) according to claim 7 or 8, wherein the distal end of the anchor holding instrument (163) consists of a hollow needle, and the anchors (27, 141) are disposed in the needle.

11. The suture instrument (161) according to any one of claims 7 to 10, further provided with an attraction instrument (136) that attracts the anchors (27, 141) with a magnetic force, wherein
the base has a lumen (134), the attraction instrument (136) is disposed along the lumen (134) of the base (162) so as to freely advance or retract, and the anchors (27, 141) and the attraction instrument (136) are both made from a magnetic body, and one of them is a magnet.

12. The suture instrument (101, 131, 151) according to claim 1,
wherein the distal end of the anchor holding instrument (112, 139, 155) is directed to the proximal end of the base (13, 102, 152) so as to be parallel to the base (13, 102, 152).

13. The suture instrument (101, 131, 151) according to claim 1,
wherein the anchor holding instrument (112, 139, 155) has a sharp distal end (112A, 139A, 163A), and
the anchor holding instrument (112, 139, 155) is capable of penetrating tissue from a distal side to a proximal side of the tissue by moving an entire suture instrument (101, 131, 151) backward in a state that the anchor holding instrument (112, 139, 155) is disposed at a position where it is separated from the base (13, 102, 152) by the tip position changing mechanism (103, 140).

## Patentansprüche

1. Nahtinstrument (101, 131, 151), das so ausgelegt ist, dass es in einen Körper eingesetzt wird und Längsverankerungen (27, 141) ausfährt, mit denen ein Nahtfaden (25) verbunden ist, umfassend:
eine Basis (13, 102, 152), die sich von einem proximalen Ende auf einer Handseite zu einem entfernten Ende erstreckt, die in ein Gewebe eingeführt wird, wobei die Basis (13, 102, 152) in der Lage ist, in eine Perforation eingesetzt zu werden, und flexibel ist;
ein Verankerungshalteinstrument (112, 139, 155), das in der Lage ist, eine Mehrzahl von Verankerungen (27, 141) im Inneren aufzunehmen, wobei das Verankerungshalteinstrument (112, 139, 155) am entfernten Ende der Base (13, 102, 152) entlang einer Achse der Base (13, 102, 152) angeordnet und aufgenommen ist, so dass das distale Ende des Verankerungshalteinstruments (112, 139, 155) ungefähr in eine Richtung eines proximalen Endes der Base (13, 102, 152) zeigt;
ein Spitzenpositionsänderungsmechanismus (103, 140), der so ausgelegt ist, dass er das distale Ende des Verankerungshalteinstruments (112, 139, 155) in eine radiale Richtung bewegt, die sich mit der Axiallinie der Basis (13, 102, 152) schneidet, von einer Position, in der das Verankerungshalteinstrument (112, 139, 155) in der Basis (13, 102, 152) aufgenommen ist, in eine Position, in der das distale Ende des Verankerungshalteinstruments (112, 139, 155) von einem distalen Ende der Basis (13, 102, 152) beabstandet ist und in die Richtung des proximalen Endes der Basis (13, 102, 152) ungefähr spitz ist; und
eine Positionsbetriebsvorrichtung (17, 121, 145, 190), die den Spitzenpositionsänderungsmechanismus (103, 140) auf der Handseite betreibt und eine Position des distalen Endes des Verankerungshalteinstruments (112, 139, 155) betreibt,
**dadurch gekennzeichnet, dass** ein distales Ende des Verankerungshalteinstruments (112, 139, 155) so ausgelegt ist, dass es in das Gewebe eingesetzt wird und die Verankerungen (27, 141) ausfährt.

2. Nahtinstrument (101, 131, 151) nach Anspruch 1, wobei der Spitzenpositionsänderungsmechanismus (103, 140) die Position dadurch ändert, dass er eine Bewegung des Verankerungshalteinstruments (112, 139, 155) in eine Position bewirkt, die gegenüber einer Axiallinie in einer Längsrichtung der Base (13, 102, 152) versetzt ist.

3. Nahtinstrument (101, 131, 151) nach Anspruch 1, wobei der Spitzenpositionsänderungsmechanismus (103, 140) die Position dadurch ändert, dass er das Verankerungshalteinstrument (112, 139, 155) in Bezug auf eine Axiallinie in einer Längsrichtung der Base (13, 102, 152) neigt.

4. Nahtinstrument (101, 131, 151) nach einem der Ansprüche 1 bis 3, wobei das Verankerungshalteinstrument (112, 139, 155) eine Nadel (112, 139, 155) ist, die am distalen Ende eine scharfe Spitze aufweist.

5. Nahtinstrument (101, 131, 151) nach einem der Ansprüche 1 bis 3, wobei das distale Ende des Verankerungshalteinstruments (112, 139, 155) aus einer Hohlnadel (112, 139, 155) besteht, und die Verankerungen (27, 141) in der Nadel (112, 139, 155) angeordnet sind.

6. Nahtinstrument (101, 131, 151) nach einem der Ansprüche 1 bis 5, das des Weiteren mit einem Anziehungsinstrument (136) bereitgestellt ist, das die Verankerungen (27, 141) mit einer Magnetkraft anzieht, wobei
die Basis ein Lumen (134) aufweist;
das Anziehungsinstrument (136) entlang des Lumens (134) der Basis (13, 102, 152) angeordnet ist, so dass es sich frei vorwärtsbewegen und zurückziehen kann, und die Verankerungen (27, 141);
und das Anziehungsinstrument (136) jeweils aus einem magnetischen Körper gebildet sind und einer davon ein Magnet ist.

7. Nahtinstrument (161), das so ausgelegt ist, dass es in einen Körper eingesetzt wird und Längsverankerungen (27, 141) ausfährt, mit denen ein Nahtfaden (25) verbunden ist, umfassend:
eine Basis (162), die sich von einem proximalen Ende auf einer Handseite zu einem entfernten Ende erstreckt, die so ausgelegt ist, dass sie in Gewebe eingeführt wird, wobei die Basis (162) in der Lage ist, in eine Perforation eingesetzt zu werden, und flexibel ist; und
ein Verankerungshalteinstrument (163), das am entfernten Ende der Basis (13, 102, 152, 162) angeordnet ist und in der Lage ist, eine Mehrzahl der Verankerungen (27, 141) im Inneren aufzunehmen,
wobei ein distales Ende des Verankerungshalteinstruments (163) so ausgelegt ist, dass es in das Gewebe eingesetzt wird und die Verankerungen (27, 141) ausfährt, wobei das distale Ende des Verankerungshalteinstruments (163) auf der Seite des entfernten Endes der Base (162) angeordnet und aufgenommen ist, wobei das Verankerungshalteinstrument (163) am entfernten Ende der Base (162) entlang einer Achse der Base (162) spitz ist;
wobei das Nahtinstrument (161) des Weiteren umfasst:
einen Umlenkmechanismus (164), der zum Umlenken einer Position des distalen Endes des Verankerungshalteinstruments (163) bereitgestellt ist und bewirkt, dass das distale Ende des Verankerungshalteinstruments (163) vom entfernten Ende der Basis (162) hin in eine Richtung umgelenkt wird, die dem proximalen Ende zugewandt ist; und
das Verankerungshalteinstrument (163) in der Lage ist, Gewebe in einem Zustand zu penetrieren, in dem die Position des distalen Endes des Verankerungshalteinstruments (163), das von der Basis (162) vorspringt, durch den Umlenkmechanismus (164) in eine Richtung vom distalen Ende der Basis (162) hin zu einem proximalen Ende der Basis (162) umgelenkt wird, und in der Lage ist, die Verankerungen (27, 141) auf einer proximalen Seite des Gewebes auszufahren,
**dadurch gekennzeichnet, dass** der Umlenkmechanismus (164) eine Mehrzahl von Krümmungsstücken (165), eine Mehrzahl von Stiften (167), die die Krümmungsstücke (165) mit benachbarten Krümmungsstücken verbinden, so dass sich diese frei um die Stifte (167) drehen, und ein Krümmungssteuerelement (168) umfasst, aus einem Formgedächtnismaterial hergestellt und in einer Linie entlang des Umlenkmechanismus (164) auf der Seite des Krümmungssteuerelements (168) angeordnet, der Umlenkmechanismus (164) so konfiguriert ist, dass er die Position des distalen Endes des Verankerungshalteinstruments (163) umlenkt, indem das Krümmungssteuerelement (168) in eine Ursprungsform zurückgebracht wird.

8. Nahtinstrument (161) nach Anspruch 7, wobei der Umlenkmechanismus (164) die Position dadurch ändert, dass er das Verankerungshalteinstrument (163) in Bezug auf eine Axiallinie in die Längsrichtung der Base (162) neigt.

9. Nahtinstrument (161) nach Anspruch 7 oder 8, wobei das Verankerungshalteinstrument (163) eine Nadel ist, die am distalen Ende eine scharfe Spitze aufweist.

10. Nahtinstrument (161) nach Anspruch 7 oder 8, wobei das distale Ende des Verankerungshalteinstruments (163) aus einer Hohlnadel besteht, und die Verankerungen (27, 141) in der Nadel angeordnet sind.

11. Nahtinstrument (161) nach einem der Ansprüche 7 bis 10, das des Weiteren mit einem Anziehungsinstrument (136) bereitgestellt ist, das die Verankerungen (27, 141) mit einer Magnetkraft anzieht, wobei
die Basis ein Lumen (134) aufweist, das Anziehungsinstrument (136) entlang des Lumens (134) der Basis (162) angeordnet ist, so dass es sich frei vorwärtsbewegen und zurückziehen kann, und die Verankerungen (27, 141) und das Anziehungsinstrument (136) jeweils aus einem magnetischen Körper gebildet sind und einer davon ein Magnet ist.

12. Nahtinstrument (101, 131, 151) nach Anspruch 1,
wobei das distale Ende des Verankerungshalteinstruments (112, 139, 155) zum proximalen Ende der Base (13, 102, 152) gerichtet ist, um parallel zur Base (13, 102, 152) zu verlaufen.

13. Nahtinstrument (101, 131, 151) nach Anspruch 1,
wobei das Verankerungshalteinstrument (112, 139, 155) ein scharfes distales Ende (112A, 139A, 136A) aufweist, und
das Verankerungshalteinstrument (112, 139, 155) in der Lage ist, Gewebe von einer distalen Seite zu einer proximalen Seite des Gewebes zu penetrieren, indem ein ganzes Nahtinstrument (101, 131, 151) in einem Zustand, in dem das Verankerungshalteinstrument (112, 139, 155) in einer Position angeordnet ist, in der es von der Basis (13, 102, 152) getrennt ist, durch den Spitzenpositionsänderungsmechanismus (103, 140) rückwärtsbewegt wird.

## Revendications

1. Instrument de suture (101, 131, 151) apte à être introduit dans un corps et à éjecter des ancrages allongés (27, 141) auxquels un fil de suture (25) est relié, comprenant :
une base (13, 102, 152) qui s'étend d'une extrémité proximale sur un côté main à une extrémité éloignée qui est introduite dans un tissu, la base (13, 102, 152) étant capable d'être introduite dans une perforation et ayant une flexibilité ;
un instrument de maintien d'ancrage (112, 139, 155) qui est capable de loger une pluralité d'ancrages (27, 141) à l'intérieur, l'instrument de maintien d'ancrage (112, 139, 155) étant disposé et logé dans l'extrémité éloignée de la base (13, 102, 152) le long d'un axe de la base (13, 102, 152) de telle sorte que l'extrémité distale de l'instrument de maintien d'ancrage (112, 139, 155) est approximativement dirigée dans une direction d'une extrémité proximale de la base (13, 102, 152) ;
un mécanisme de changement de position de pointe (103, 140) apte à déplacer l'extrémité distale de l'instrument de maintien d'ancrage (112, 139, 155) dans une direction radiale coupant la ligne axiale de la base (13, 102, 152) d'une position dans laquelle l'instrument de maintien d'ancrage (112, 139, 155) est logé dans la base (13, 102, 152) à une position dans laquelle l'extrémité distale de l'instrument de maintien d'ancrage (112, 139, 155) est espacée d'une extrémité distale de la base (13, 102, 152) et est approximativement dirigée dans la direction de l'extrémité proximale de la base (13, 102, 152) ; et
un dispositif d'actionnement de position (17, 121, 145, 190) qui actionne le mécanisme de changement de position de pointe (103, 140) sur le côté main et actionne une position de l'extrémité distale de l'instrument de maintien d'ancrage (112, 139, 155),
**caractérisé par le fait qu'**une extrémité distale de l'instrument de maintien d'ancrage (112, 139, 155) est apte à être introduite dans le tissu et à éjecter les ancrages (27, 141).

2. Instrument de suture (101, 131, 151) selon la revendication 1, dans lequel le mécanisme de changement de position de pointe (103, 140) change la position en amenant l'instrument de maintien d'ancrage (112, 139, 155) à se déplacer à une position qui est décalée par rapport à une ligne axiale dans une direction longitudinale de la base (13, 102, 152).

3. Instrument de suture (101, 131, 151) selon la revendication 1, dans lequel le mécanisme de changement de position de pointe (103, 140) change la position en inclinant l'instrument de maintien d'ancrage (112, 139, 155) par rapport à une ligne axiale dans une direction longitudinale de la base (13, 102, 152).

4. Instrument de suture (101, 131, 151) selon l'une quelconque des revendications 1 à 3, dans lequel l'instrument de maintien d'ancrage (112, 139, 155) est une aiguille (112, 139, 155) qui a à l'extrémité distale une pointe acérée.

5. Instrument de suture (101, 131, 151) selon l'une quelconque des revendications 1 à 3, dans lequel l'extrémité distale de l'instrument de maintien d'ancrage (112, 139, 155) consiste en une aiguille creuse (112, 139, 155), et les ancrages (27, 141) sont disposés dans l'aiguille (112, 139, 155).

6. Instrument de suture (101, 131, 151) selon l'une quelconque des revendications 1 à 5, comprenant en outre un instrument d'attraction (136) qui attire les ancrages (27, 141) avec une force magnétique, dans lequel :
la base a une lumière (134) ;
l'instrument d'attraction (136) est disposé le long de la lumière (134) de la base (13, 102, 152) de façon à avancer et se rétracter librement, et les ancrages (27, 141) ;
et l'instrument d'attraction (136) sont tous les deux réalisés à partir d'un corps magnétique, et l'un d'eux est un aimant.

7. Instrument de suture (161) apte à être introduit dans un corps et à éjecter des ancrages allongés (27, 141) auxquels un fil de suture (25) est relié, comprenant:
une base (162) qui s'étend d'une extrémité proximale sur un côté main à une extrémité éloignée apte à être introduite dans un tissu, la base (162) étant capable d'être introduite dans une perforation et ayant une flexibilité ; et
un instrument de maintien d'ancrage (163) qui est disposé à l'extrémité éloignée de la base (13, 102, 152, 162) et est capable de loger une pluralité d'ancrages (27, 141) à l'intérieur,
une extrémité distale de l'instrument de maintien d'ancrage (163) est apte à être introduite dans le tissu et à éjecter les ancrages (27, 141), l'extrémité distale de l'instrument de maintien d'ancrage (163) étant disposée et logée dans le côté d'extrémité éloignée de la base (162), l'instrument de maintien d'ancrage (163) étant dirigé dans l'extrémité éloignée de la base (162) le long d'un axe de la base (162) ;
l'instrument de suture (161) comprenant en outre :
un mécanisme de déviation (164) qui est prévu pour dévier une position de l'extrémité distale de l'instrument de maintien d'ancrage (163) et amène l'extrémité distale de l'instrument de maintien d'ancrage (163) à dévier depuis l'extrémité éloignée de la base (162) vers une direction qui fait face à l'extrémité proximale ; et
l'instrument de maintien d'ancrage (163) est capable de pénétrer dans un tissu dans un état dans lequel la position de l'extrémité distale de l'instrument de maintien d'ancrage (163) qui fait saillie à partir de la base (162) est déviée dans une direction allant de l'extrémité distale de la base (162) vers une extrémité proximale de la base (162) par le mécanisme de déviation (164) et est capable d'éjecter les ancrages (27, 141) à un côté proximal du tissu,
**caractérisé par le fait que** le mécanisme de déviation (164) comprend une pluralité de pièces d'incurvation (165), une pluralité d'axes (167) reliant les pièces d'incurvation (165) à des pièces d'incurvation adjacentes de façon à tourner librement autour des axes (167), et un élément de commande d'incurvation (168) fait de matériau à mémoire de forme et disposé dans une ligne le long du mécanisme de déviation (164) sur le côté de l'élément de commande d'incurvation (168), le mécanisme de déviation (164) est configuré pour dévier la position de l'extrémité distale de l'instrument de maintien d'ancrage (163) par l'élément de commande d'incurvation (168) qui est ramené à une forme initiale.

8. Instrument de suture (161) selon la revendication 7, dans lequel le mécanisme de déviation (164) change la position en inclinant l'instrument de maintien d'ancrage (163) par rapport à une ligne axiale dans la direction longitudinale de la base (162).

9. Instrument de suture (161) selon la revendication 7 ou 8, dans lequel l'instrument de maintien d'ancrage (163) est une aiguille qui a à l'extrémité distale une pointe acérée.

10. Instrument de suture (161) selon la revendication 7 ou 8, dans lequel l'extrémité distale de l'instrument de maintien d'ancrage (163) consiste en une aiguille creuse, et les ancrages (27, 141) sont disposés dans l'aiguille.

11. Instrument de suture (161) selon l'une quelconque des revendications 7 à 10, comprenant en outre un instrument d'attraction (136) qui attire les ancrages (27, 141) avec une force magnétique, dans lequel :
la base a une lumière (134), l'instrument d'attraction (136) est disposé le long de la lumière (134) de la base (162) de façon à avancer ou se rétracter librement, et les ancrages (27, 141) et l'instrument d'attraction (136) sont tous les deux réalisés à partir d'un corps magnétique, et l'un d'eux est un aimant.

12. Instrument de suture (101, 131, 151) selon la revendication 1, dans lequel l'extrémité distale de l'instrument de maintien d'ancrage (112, 139, 155) est dirigée vers l'extrémité proximale de la base (13, 102, 152) de façon à être parallèle à la base (13, 102, 152).

13. Instrument de suture (101, 131, 151) selon la revendication 1, dans lequel l'instrument de maintien d'ancrage (112, 139, 155) a une extrémité distale acérée (112A, 139A, 136A), et
l'instrument de maintien d'ancrage (112, 139, 155) est capable de pénétrer dans un tissu d'un côté distal à un côté proximal du tissu en déplaçant un instrument de suture entier (101, 131, 151) vers l'arrière dans un état dans lequel l'instrument de maintien d'ancrage (112, 139 155) est disposé à une position dans laquelle il est séparé de la base (13, 102, 152) par le mécanisme de changement de position de pointe (103, 140).
